# EUROPÄISCHE PATENTSCHRIFT

(11) **EP 1 869 141 B1**
(45) Veröffentlichungstag und Bekanntmachung des Hinweises auf die Patenterteilung: **11.03.2020**
(21) Anmeldenummer: 06723550.7
(22) Anmeldetag: 20.03.2006
(51) Int. Cl.: C09K 11/06, C07C 211/61, C07C 39/17, C07F 9/50, C07F 9/53, C07C 17/12, C07C 25/22, C09B 3/78, H01L 51/00, C07C 205/06, C07F 9/92, C09B 57/00, H05B 33/14, C07C 43/275, C07C 49/792

(54) **VERBINDUNGEN FÜR ORGANISCHE ELEKTRONISCHE VORRICHTUNGEN**
COMPOUNDS FOR ORGANIC ELECTRONIC DEVICES
COMPOSÉS POUR DISPOSITIFS ELECTRONIQUES ORGANIQUES

(30) Priorität: 14.04.2005 EP 05008130
(43) Veröffentlichungstag der Anmeldung: 26.12.2007
(73) Patentinhaber: Merck Patent GmbH, 64293 Darmstadt (DE)
(72) Erfinder: HEIL, Holger, 64285 Darmstadt (DE); STOESSEL, Philipp, 60487 Frankfurt/Main (DE); VESTWEBER, Horst, 34630 Gilersberg-Winterscheid (DE); FORTTE, Rocco, 65933 Frankfurt/Main (DE)
(86) Internationale Anmeldenummer: PCT/EP2006/002531
(87) Internationale Veröffentlichungsnummer: WO 2006/108497

(56) Entgegenhaltungen:
- EP-A- 1 061 112
- EP-A- 1 221 434
- WO-A-2004/020387
- WO-A-2004/061047
- JP-A- H11 144 875
- US-A1- 2003 072 966
- US-A1- 2004 253 389
- PATENT ABSTRACTS OF JAPAN Bd. 2003, Nr. 12, 5. Dezember 2003 (2003-12-05) & JP 2003 238516 A (PETROLEUM ENERGY CENTER; IDEMITSU KOSAN CO LTD), 27. August 2003 (2003-08-27)
- KANNAN R: "Toward Highly Active Two-Photon Absorbing Liquids. Synthesis and Characterization of 1,3,5-Triazine-Based Octupolar Molecules" CHEMISTRY OF MATERIALS, Bd. 16, Nr. 1, 2004, Seiten 185-194, XP002350518
- DATABASE WPI Section Ch, Week 199938 Derwent Publications Ltd., London, GB; Class E14, AN 1999-448144 XP002350532 & JP 11 184109 A (CANON KK) 9. Juli 1999 (1999-07-09) & JP 11 184109 A (CANON INC) 9. Juli 1999 (1999-07-09)

## Beschreibung

Die vorliegende Erfindung beschreibt neue Verbindungen und deren Einsatz in organischen elektronischen Vorrichtungen.

Der Einsatz halbleitender organischer Verbindungen, die zur Emission von Licht im sichtbaren Spektralbereich befähigt sind, in organischen Elektrolumineszenzvorrichtungen (OLEDs) ist bekannt. Der allgemeine Aufbau derartiger Vorrichtungen ist beispielsweise in US 4539507, US 5151629, EP 0676461 und WO 98/27136 beschrieben.

Allerdings zeigen diese Vorrichtungen immer noch erhebliche Probleme, die einer dringenden Verbesserung bedürfen:
1. Die Effizienz ist gerade bei fluoreszierenden OLEDs immer noch zu niedrig und muss verbessert werden.
2. Die operative Lebensdauer ist insbesondere bei blauer Emission immer noch gering, so dass bisher nur einfache Anwendungen kommerziell realisiert werden konnten.
3. Die Betriebsspannung ist gerade bei fluoreszierenden OLEDs recht hoch und sollte daher weiter verringert werden, um die Leistungseffizienz zu verbessern. Das ist insbesondere für mobile Anwendungen von großer Bedeutung. Insbesondere zeigen OLEDs gemäß dem Stand der Technik eine starke Abhängigkeit der Betriebsspannung von der Schichtdicke der Lochtransportschicht.
4. Viele blau emittierende Emitter, die sowohl aromatische Amine, wie auch Doppelbindungssysteme enthalten, sind thermisch nicht stabil und zersetzen sich beim Sublimieren oder beim Aufdampfen. Dadurch ist die Verwendung dieser Systeme nicht bzw. nur unter großen Verlusten und mit hohem technischen Aufwand möglich.

In WO 2004/061047 wird die Verwendung von Benzofluorenyl-Aminen in OLEDs offenbart. In JP 2003/238516 sind Arylamine zur Verwendung in OLEDs offenbart, die ein großes kondensiertes aromatisches System aufweisen. In Kannan et al., Chem. Mater. 2004, 16, 185 ff. sind Synthese und Eigenschaften von bestimmten Fluorenylaminen offenbart.

Als nächstliegender Stand der Technik kann die Verwendung bestimmter Arylvinylamine von Idemitsu genannt werden (z. B. WO 04/013073, WO 04/016575, WO 04/018587). Damit werden sehr gute Lebensdauern bei tiefblauer Emission zitiert. Allerdings sind diese Ergebnisse stark abhängig vom verwendeten Hostmaterial, so dass die zitierten Lebensdauern nicht als Absolutwerte verglichen werden können, sondern immer nur bei Einsatz in einem optimierten System. Weiterhin sind diese Verbindungen thermisch instabil und lassen sich nicht unzersetzt verdampfen, was daher einen hohen technischen Aufwand für die OLED-Herstellung erfordert und somit einen deutlichen technischen Nachteil darstellt. Einen weiteren Nachteil stellt die Emissionsfarbe dieser Verbindungen dar. Während Idemitsu tiefblaue Emission (CIE-y-Koordinaten im Bereich von 0.15-0.18) zitiert, konnten diese Farbkoordinaten nicht in einfachen Vorrichtungen gemäß dem Stand der Technik reproduziert werden. Im Gegenteil erhält man hier grünblaue Emission. Es ist nicht offensichtlich, wie mit diesen Verbindungen tatsächlich blaue Emission erzeugt werden kann.

Es besteht also weiterhin ein Bedarf an blau emittierenden Verbindungen, die in organischen Elektrolumineszenzvorrichtungen zu guten Effizienzen und gleichzeitig zu hohen Lebensdauern führen und die technisch unproblematisch zu verarbeiten sind. Es wurde nun überraschend gefunden, dass organische Elektrolumineszenzvorrichtungen, die bestimmte - im Folgenden aufgeführte - Verbindungen als blau emittierende Dotanden in einem Hostmaterial enthalten, deutliche Verbesserungen gegenüber dem Stand der Technik aufweisen. Mit diesen Materialien ist es möglich, längere Lebensdauern bei höherer Effizienz zu erhalten. Außerdem lassen sich diese Verbindungen im Gegensatz zu Materialien gemäß dem Stand der Technik ohne merkliche Zersetzung auch in größeren Mengen sublimieren und sind daher deutlich leichter zu handhaben als Materialien gemäß dem Stand der Technik.
Weiterhin eignen sich diese Verbindungen für die Verwendung als Lochtransportmaterial, als Elektronentransportmaterial oder als Matrixmaterial für phosphoreszierende Vorrichtungen. Diese Verbindungen und deren Verwendung in OLEDs sind daher Gegenstand der vorliegenden Erfindung.

Gegenstand der Erfindung sind Verbindungen gemäß Formel (1), wie definiert in Anspruch 1.

Unter einer Arylgruppe bzw. einer Heteroarylgruppe im Sinne dieser Erfindung wird eine aromatische Gruppe bzw. heteroaromatische Gruppe mit einem gemeinsamen aromatischen Elektronensystem verstanden, wobei eine Arylgruppe 6 bis 24 C-Atome und eine Heteroarylgruppe 2 bis 24 C-Atome und insgesamt mindestens 5 aromatische Ringatome umfasst. Die Heteroatome sind bevorzugt ausgewählt aus N, O und/oder S. Dies kann im Sinne dieser Erfindung ein einfacher Homo- oder Heterocyclus sein, beispielsweise Benzol, Pyridin, Thiophen, etc., oder es kann ein kondensiertes aromatisches Ringsystem sein, in dem mindestens zwei aromatische oder heteroaromatische Ringe, beispielsweise Benzolringe, miteinander "verschmolzen", d. h. durch Anellierung einander ankondensiert sind, also mindestens eine gemeinsame Kante und dadurch auch ein gemeinsames aromatisches System aufweisen. Diese Aryl- oder Heteroarylgruppe kann substituiert oder unsubstituiert sein; ebenso können gegebenenfalls vorhandene Substituenten weitere Ringsysteme bilden. So sind beispielsweise Systeme wie Naphthalin, Anthracen, Phenanthren, Pyren, etc. als Arylgruppen und Chinolin, Acridin, Benzothiophen, Carbazol, etc. als Heteroarylgruppen im Sinne dieser Erfindung zu sehen, während beispielsweise Biphenyl, Fluoren, Spirobifluoren, etc. keine Arylgruppen darstellen, da es sich hierbei um separate aromatische Elektronensysteme handelt.

Ein aromatisches Ringsystem im Sinne dieser Erfindung enthält 6 bis 40 C-Atome im Ringsystem. Ein heteroaromatisches Ringsystem im Sinne dieser Erfindung enthält 2 bis 40 C-Atome und mindestens ein Heteroatom im Ringsystem, mit der Maßgabe, dass die Summe der C-Atome und Heteroatome mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (weniger als 10 % der von H verschiedenen Atome, bevorzugt weniger als 5 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, etc. als aromatische Ringsysteme im Sinne dieser Erfindung verstanden werden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Ethoxy, n-Propoxy, i-Propoxy, n-Butoxy, i-Butoxy, s-Butoxy, t-Butoxy oder 2-Methylbutoxy verstanden. Unter einer C₂-C₂₄ Aryl- oder Heteroarylgruppe, die je nach Verwendung monovalent oder bivalent sein kann, die noch jeweils mit den oben genannten Resten R¹ substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol. Unter aromatischen und heteroaromatischen Ringsystemen im Sinne dieser Erfindung werden außer den oben genannten Aryl- und Heteroarylgruppen beispielsweise Biphenylen, Terphenylen, Fluoren, Spirobifluoren, Dihydrophenanthren, Tetrahydropyren und cis- oder trans-Indenofluoren verstanden.

Bevorzugt sind Verbindungen gemäß Formel (1), in denen das Symbol Y für Stickstoff, Phosphor, C=O oder P=O steht, besonders bevorzugt für Stickstoff, C=O oder P=O, ganz besonders bevorzugt für Stickstoff. Dabei hängt die Wahl der Einheit Y von der gewünschten Funktion der Verbindung gemäß Formel (1) ab. Soll die Verbindung der Formel (1) als Emitter in einer Emissionsschicht oder als Lochtransportmaterial, beispielsweise in einer Lochtransport- oder Lochinjektionsschicht, eingesetzt werden, so steht das Symbol Y bevorzugt für Stickstoff oder Phosphor, besonders bevorzugt für Stickstoff. Soll die Verbindung der Formel (1) als Matrixmaterial für phosphoreszierende Emitter in einer Emissionsschicht oder als Elektronentransport- und/oder Lochblockiermaterial, beispielsweise in einer Elektronentransportschicht oder in einer Lochblockierschicht, für phosphoreszierende oder fluoreszierende Vorrichtungen eingesetzt werden, so steht das Symbol Y bevorzugt für C=O oder für P=O.

Besonders bevorzugt sind also Verbindungen gemäß Formel (2), 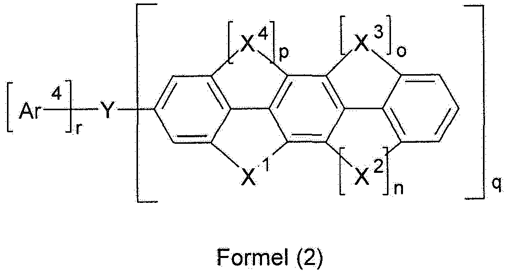 wobei die verwendeten Symbole und Indizes dieselbe Bedeutung haben, wie oben beschrieben, und wobei jede der Phenyl- bzw. Phenylengruppen noch durch einen oder zwei Reste R¹ substituiert sein kann.

Wenn die Phenyl- bzw. Phenylengruppen der Formel (2) durch R¹ substituiert sind, so sind die Reste R¹ bevorzugt an der Position gebunden, wie in Formel (2a) gezeigt:

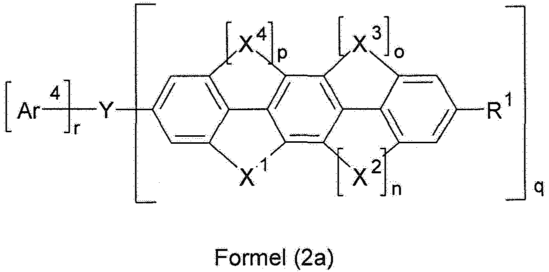

Bevorzugt sind weiterhin Verbindungen gemäß Formel (1), in denen das Symbol Ar⁴ gleich oder verschieden bei jedem Auftreten für ein aromatisches oder heteroaromatisches Ringsystem mit 5-16 aromatischen Ringatomen oder für Spirobifluoren steht, das jeweils mit einem oder mehreren Resten R¹ substituiert sein kann, besonders bevorzugt für ein aromatisches oder heteroaromatisches Ringsystem, ausgewählt aus Benzol, Naphthalin, Anthracen, Phenanthren, Pyridin, Pyren und Thiophen, insbesondere Benzol, das jeweils mit einem oder zwei Resten R¹ substituiert sein kann.

Bevorzugt sind weiterhin Verbindungen gemäß Formel (1), in denen das Symbol R¹ gleich oder verschieden bei jedem Auftreten für H, F, CN, Si(R²)₃, eine geradkettige Alkylgruppe mit 1 bis 5 C-Atomen oder eine verzweigte Alkylgruppe mit 3 bis 5 C-Atomen, wobei eine oder mehrere nicht benachbarte CH₂-Gruppen durch Si(R²)₂, -R²C=CR²-, -C=C-, -O- oder -S- ersetzt sein können und wobei ein oder mehrere H-Atome durch F ersetzt sein können, oder eine monovalente Aryl- oder Heteroarylgruppe mit 5 bis 16 aromatischen Ringatomen, die mit einem oder mehreren Resten R² substituiert sein kann, steht, besonders bevorzugt für H, F, CN, Si(Me)₃, Methyl, tert-Butyl, eine Phenylgruppe oder eine monovalente Heteroarylgruppe mit 5 oder 6 aromatischen Ringatomen, die jeweils mit einem oder mehreren Resten R² substituiert sein kann. Ganz besonders bevorzugt ist R¹ = H, wenn es direkt an eine der Gruppen Ar¹ bis Ar⁴ gebunden ist. Besonders bevorzugt ist R¹, wenn es an die Gruppe X¹, X², X³ und/oder X⁴ gebunden ist, gleich oder verschieden bei jedem Auftreten Methyl, tert-Butyl, eine Phenylgruppe, welche durch eine oder mehrere C₁- bis C₄-Alkylgruppen substituiert sein kann, oder eine monovalente Heteroarylgruppe mit 5 oder 6 aromatischen Ringatomen, welche durch eine oder mehrere C₁- bis C₄-Alkylgruppen substituiert sein kann, ganz besonders bevorzugt Methyl oder eine Phenylgruppe, welche durch eine oder mehrere C₁- bis C₄-Alkylgruppen substituiert sein kann. Dabei können jeweils auch zwei oder mehrere Reste R¹ miteinander ein Ringsystem bilden.

Bevorzugt sind weiterhin Verbindungen gemäß Formel (1), in denen die Symbole X¹, X², X³ und X⁴ bei jedem Auftreten gleich oder verschieden eine Brücke sind, die mit Ar¹ und Ar² bzw. mit Ar² und Ar³ ein cyclisches System aufspannt, ausgewählt aus C(R¹)₂, C=O, C=NR¹, O, S, S=O, SO₂, N(R¹), P(R¹), P(=O)R¹, C(R¹)₂-C(R¹)₂, C(R¹)₂-C(R¹)₂-C(R¹)₂, C(R¹)₂-O, C(R¹)₂-O-C(R¹)₂. Besonders bevorzugt sind Verbindungen gemäß Formel (1), in denen die Symbole X¹, X², X³ und X⁴ bei jedem Auftreten gleich oder verschieden ausgewählt sind aus C(R¹)₂, N(R¹), P(R¹) und P(=O)(R¹). Ganz besonders bevorzugt ist C(R¹)₂.

Bevorzugt sind weiterhin Verbindungen, in denen p = 0 ist und einer der beiden Indizes n oder o = 1 ist, während der andere der beiden Indizes = 0 ist; besonders bevorzugt sind p und n = 0 und o = 1.

Besonders bevorzugt sind also Verbindungen gemäß Formel (3) bzw. gemäß Formel (4), 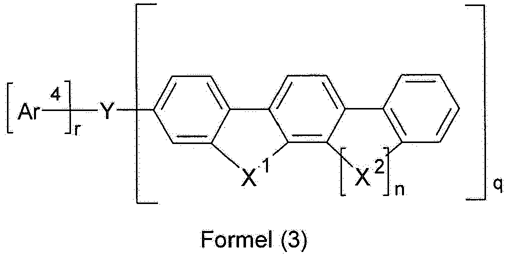 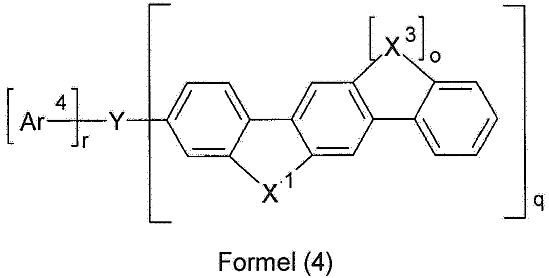 wobei die verwendeten Symbole und Indizes dieselbe Bedeutung haben, wie oben beschrieben, und wobei jede der Phenyl- bzw. Phenylengruppen noch durch einen oder zwei Reste R¹ substituiert sein kann.

Bevorzugt sind weiterhin Verbindungen, in denen der Index q gleich oder verschieden bei jedem Auftreten für 2 oder 3 steht, ganz besonders bevorzugt für 3, wenn Y aus der fünften Hauptgruppe ausgewählt ist. Wenn Y aus der sechsten Hauptgruppe ausgewählt ist, ist der Index q bevorzugt gleich 2.

Besonders bevorzugt sind Verbindungen gemäß Formel (1), die symmetrisch aufgebaut sind und die eine dreizählige Drehachse aufweisen, wenn Y aus der fünften Hauptgruppe ausgewählt ist, bzw. eine zweizählige Drehachse aufweisen, wenn Y aus der sechsten Hauptgruppe ausgewählt ist, was sich nicht nur auf die aromatischen Gruppen Ar¹ bis Ar³ bezieht, sondern auch auf die Brücken X¹ bis X⁴ und die Reste R¹ und R².

Beispiele für bevorzugte Verbindungen gemäß Formel (1) sind die im Folgenden abgebildeten Strukturen.

| | |
|---|---|
| 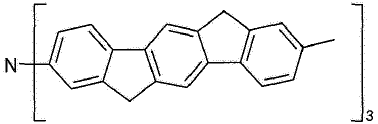 | 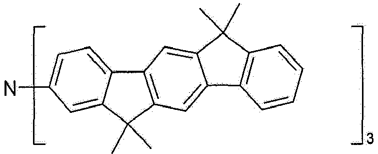 |
| (1) | (2) |
| 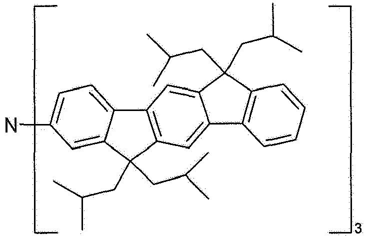 | 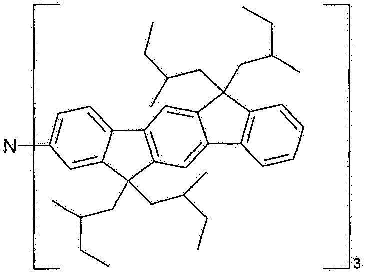 |
| (3) | (4) |
| 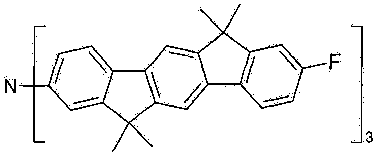 | 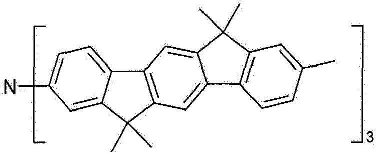 |
| (5) | (6) |
| 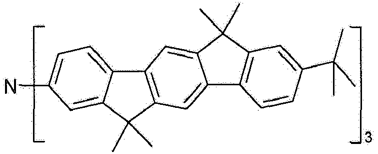 | 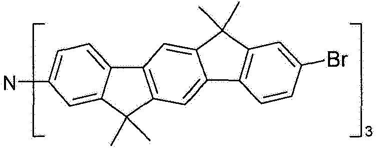 |
| (7) | (8) |
| 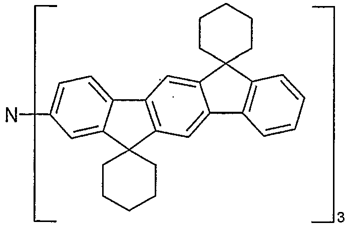 | 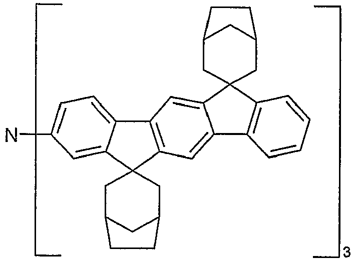 |
| (9) | (10) |
| 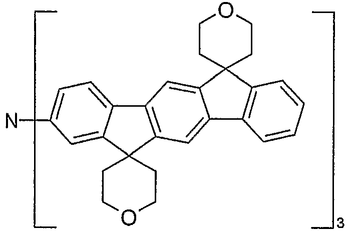 | 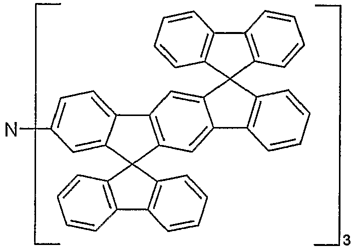 |
| (11) | (12) |
| 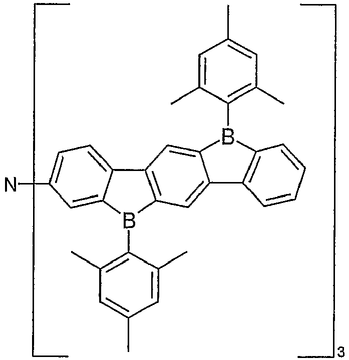 | 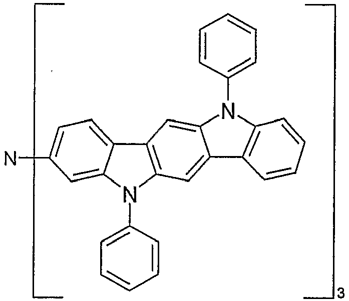 |
| (13) | (14) |
| 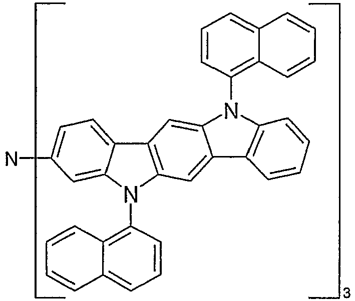 | 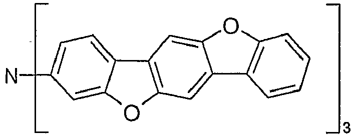 |
| (15) | (16) |
| 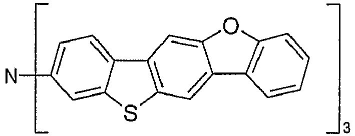 | 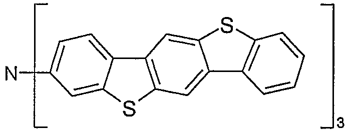 |
| (17) | (18) |
| 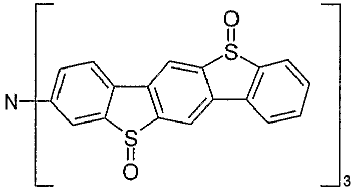 | 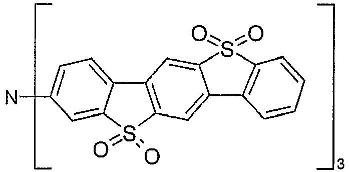 |
| (19) | (20) |
| 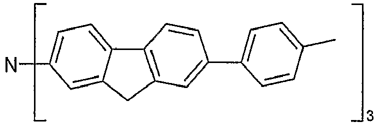 | 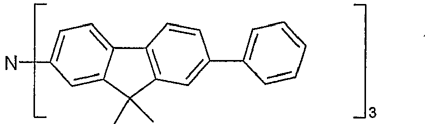 |
| (21) | (22) |
| 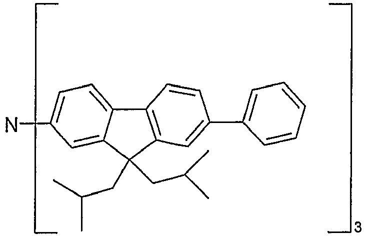 | 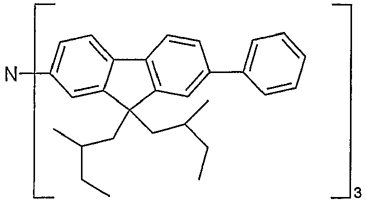 |
| (23) | (24) |
| 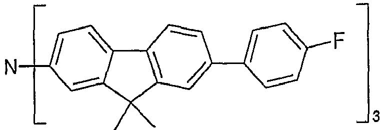 | 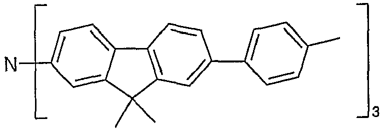 |
| (25) | (26) |
| 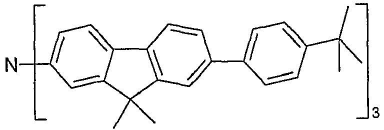 | 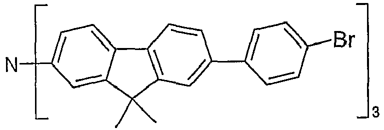 |
| (27) | (28) |
| 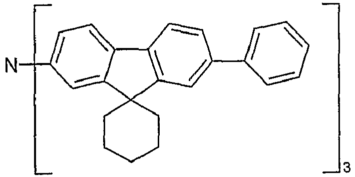 | 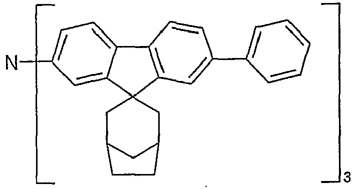 |
| (29) | (30) |
| 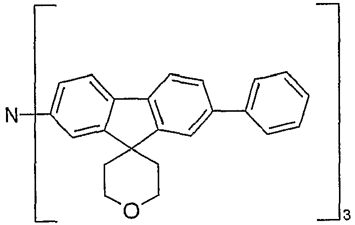 | 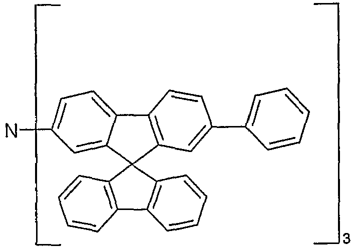 |
| (31) | (32) |
| 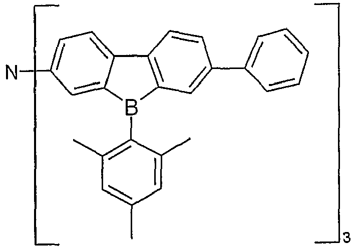 | 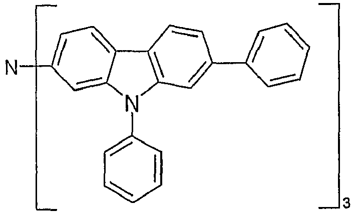 |
| (33) | (34) |
| 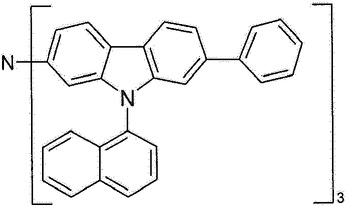 | 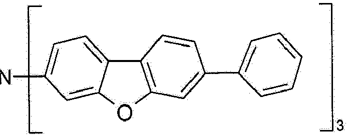 |
| (35) | (36) |
| 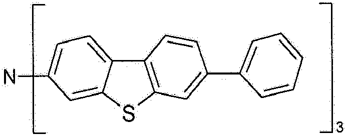 | 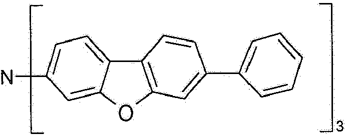 |
| (37) | (38) |
| 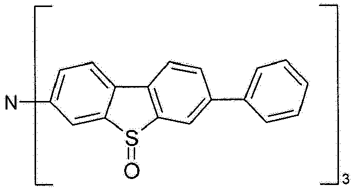 | 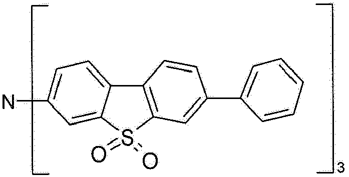 |
| (39) | (40) |
| 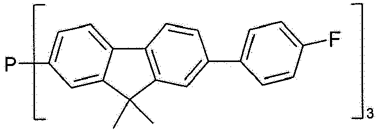 | 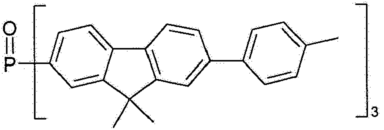 |
| (45) | (46) |
| 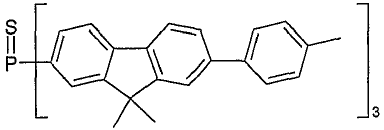 | 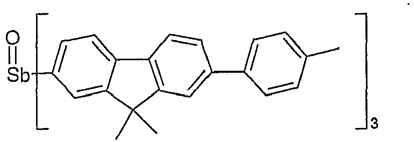 |
| (47) | (48) |
| 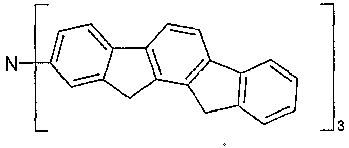 | 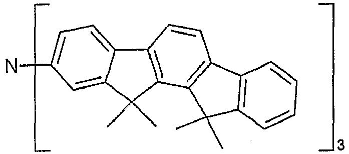 |
| (49) | (50) |
| 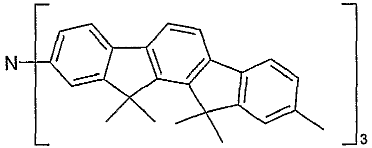 | 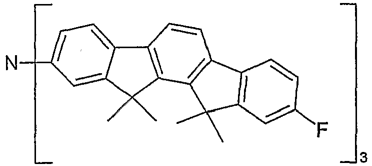 |
| (51) | (52) |
| 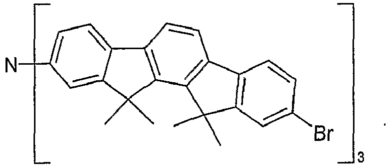 | 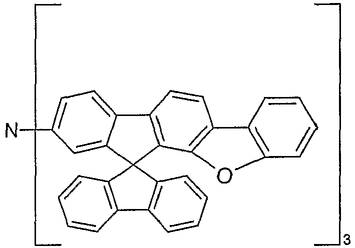 |
| (53) | (54) |
| 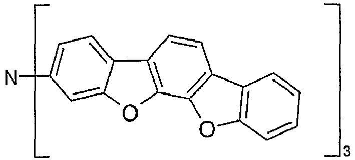 | 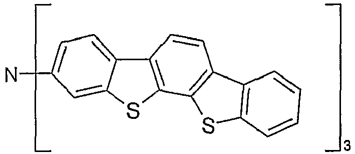 |
| (55) | (56) |
| 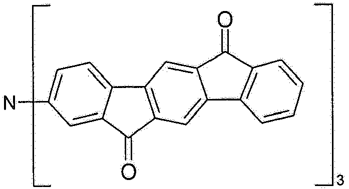 | 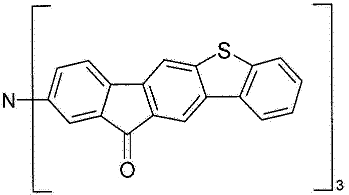 |
| (57) | (58) |
| 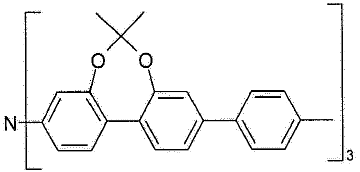 | 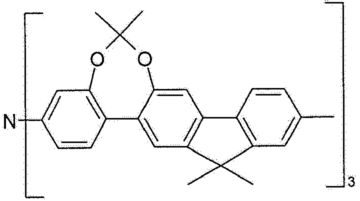 |
| (59) | (60) |
| 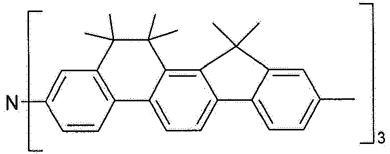 | 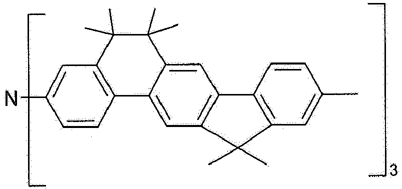 |
| (61) | (62) |
| 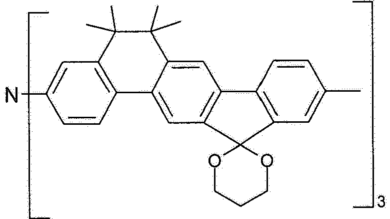 | 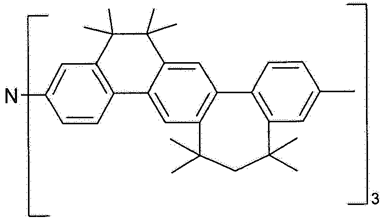 |
| (63) | (64) |
| 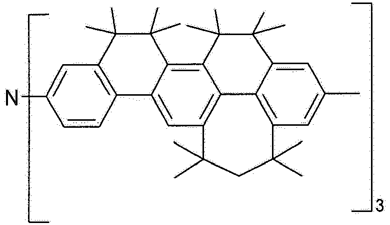 | |
| (65) | |
| 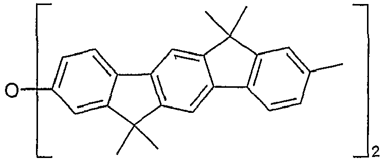 | 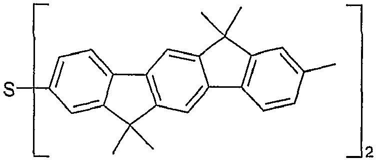 |
| (77) | (78) |
| 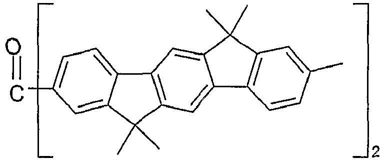 | 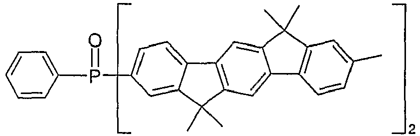 |
| (79) | (80) |
| 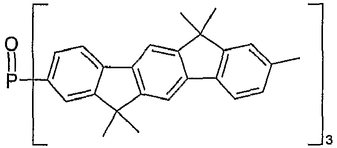 | 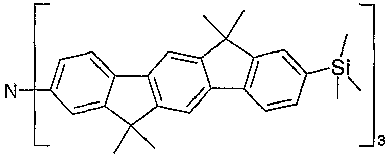 |
| (81) | (82) |

Die oben beschriebenen erfindungsgemäßen Verbindungen, z. B. Verbindungen gemäß den Strukturen (8), (28) und (53), können beispielsweise als Comonomere zur Erzeugung entsprechender konjugierter, teilkonjugierter oder nicht-konjugierter Polymere, Oligomere oder auch als Kern von Dendrimeren Verwendung finden. Die Polymerisation erfolgt dabei bevorzugt über die Halogenfunktionalität.

So können sie u. a. in lösliche Polyfluorene (z. B. gemäß EP 842208 oder WO 00/22026), Poly-spirobifluorene (z. B. gemäß EP 707020, EP 894107 oder EP 04028865.6), Poly-para-phenylene (z. B. gemäß WO 92/18552), Poly-carbazole (z. B. gemäß WO 04/070772 und WO 04/113468), Polyvinylcarbazole, Polythiophene (z. B. gemäß EP 1028136), Polydihydrophenanthrene (z. B. gemäß WO 05/014689), Polyindenofluorene (z. B. gemäß WO 04/041901 und WO 04/113412), Polyketone (z. B. gemäß WO 05/040302) oder auch in Copolymere aus mehreren dieser Einheiten einpolymerisiert werden.

Weiterer Gegenstand der Erfindung sind somit konjugierte, teilkonjugierte und nicht-konjugierte Polymere, Oligomere oder Dendrimere enthaltend eine oder mehrere Verbindungen gemäß Formel (1), wobei eine oder mehrere Bindungen der Verbindung gemäß Formel (1) zum Polymer oder Dendrimer vorhanden sind.

Die erfindungsgemäßen Verbindungen können nach dem Fachmann bekannten Syntheseschritten, wie z. B. Bromierung, Suzuki-Kupplung, Hartwig-Buchwald-Kupplung, etc., dargestellt werden.

So können die Indenofluoren-Vorstufen beispielsweise hergestellt werden, wie in Syntheseschema 1 abgebildet: Durch Suzuki-Kupplung einer Benzolboronsäure und 1,4-Dibrom-2,5-bis(methylcarboxylat)benzol, gefolgt von Ringschluss unter Einwirkung einer starken Säure und Reduktion ist das unsubstituierte trans-Indenofluoren erhältlich, welches mit Alkylierungsmitteln alkyliert werden kann. Dieses kann durch stöchiometrische Reaktion mit einem Bromierungsmittel selektiv monobromiert werden bzw. durch Nitrierung und Reduktion in die entsprechende Aminoverbindung umgewandelt werden. Die Synthese von Tris(indenofluorenyl)amin kann durch Hartwig-Buchwald-Kupplung der Monobrom- und der Aminoverbindung erfolgen, wie in Syntheseschema 2 dargestellt. Ebenso können unsymmetrische Bis(indenofluorenyl)arylamine durch Hartwig-Buchwald-Kupplung dargestellt werden, wie in Syntheseschema 3 dargestellt. Die Synthese von Tris(indenofluorenyl)phosphinen bzw. -phosphinoxiden kann aus Monobromindenofluoren durch Lithiierung und Umsetzung mit PCl₃ erfolgen, wie in Syntheseschema 4 dargestellt. Oxidation ergibt dann das entsprechende Phosphinoxid. Ebenso lassen sich hier andere Elektrophile einsetzen, wie z. B. AsCl₃, ArylPCl₂, SOCl₂, Ar₂S₂, etc. Weitere erfindungsgemäße Verbindungen können nach diesen und ähnlichen Syntheseschemata gemäß Verfahren, die dem Fachmann für organische Synthese bekannt sind, synthetisiert werden. Weiterhin können sich die erhaltenen Verbindungen nach Standardverfahren bromieren lassen und können so als Monomere für Polymere, Oligomere oder Dendrimere eingesetzt werden.

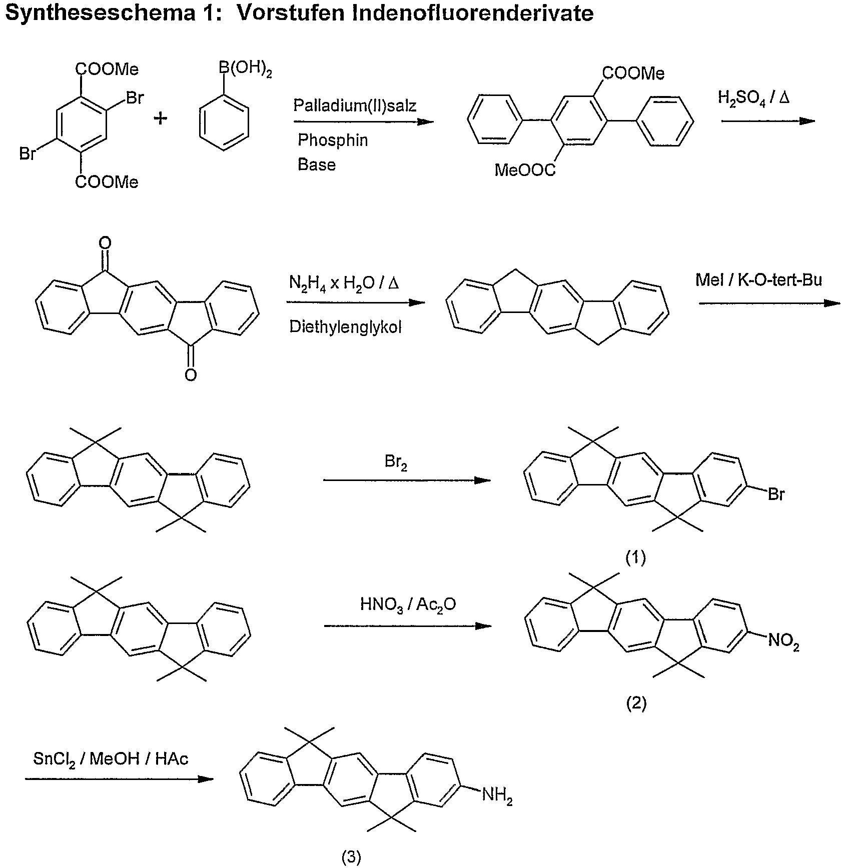

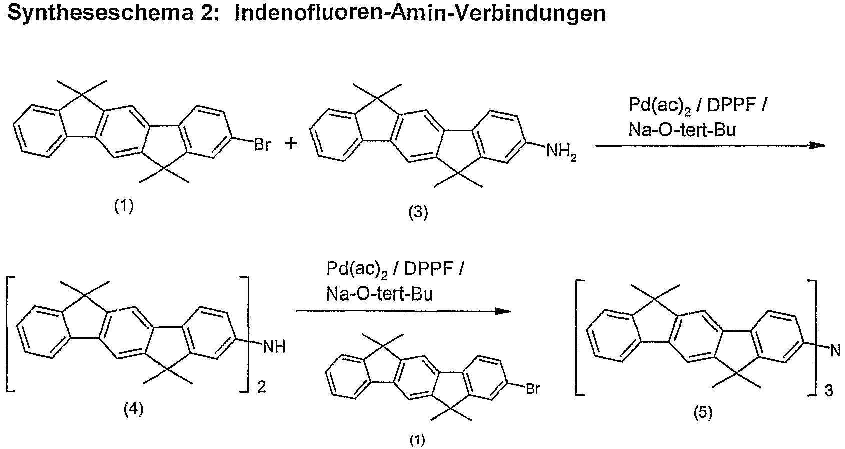

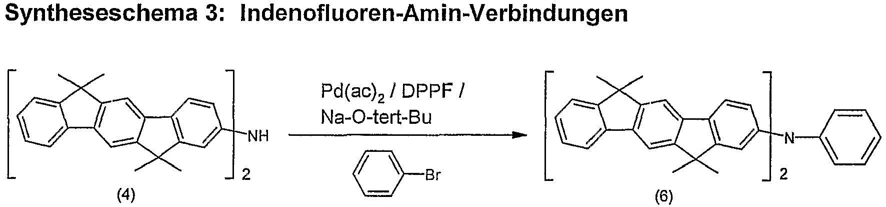

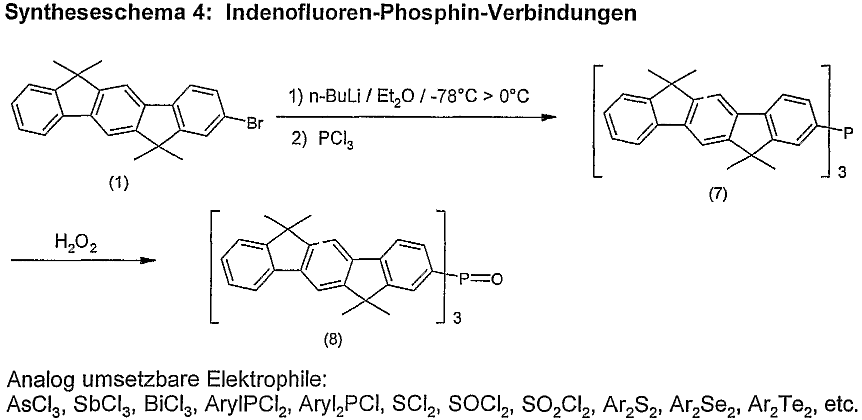

Die Verbindungen gemäß Formel (1) können in organischen Elektrolumineszenzvorrichtungen eingesetzt werden. Dabei wird die Verbindung bevorzugt in der emittierenden Schicht als Mischung mit mindestens einem Hostmaterial eingesetzt. Es ist bevorzugt, wenn die Verbindung gemäß Formel (1) in der Mischung die emittierende Verbindung (der Dotand) ist. Bevorzugte Hostmaterialien sind organische Verbindungen, deren Emission kürzerwellig ist als die der Verbindung gemäß Formel (1) oder die überhaupt nicht emittieren.

Gegenstand der Erfindung sind daher weiterhin Mischungen enthaltend mindestens eine Verbindung gemäß Formel (1) und mindestens ein Hostmaterial.

Wenn die Verbindung der Formel (1) als emittierender Dotand eingesetzt wird, kommen als Hostmaterialien verschiedene Stoffklassen in Frage. Bevorzugte Hostmaterialien sind ausgewählt aus den Klassen der Oligo-arylene (z. B. 2,2',7,7'-tetraphenyl-spirobifluoren gemäß EP 676461 oder Dinaphthylanthracen), insbesondere der Oligo-arylene enthaltend kondensierte aromatische Gruppen, der Oligo-arylenvinylene (z. B. DPVBi oder Spiro-DPVBi gemäß EP 676461), der polypodalen Metallkomplexe (z. B. gemäß WO 04/081017), der lochleitenden Verbindungen (z. B. gemäß WO 04/058911), der elektronenleitenden Verbindungen, insbesondere Ketone, Phosphinoxide, Sulfoxide, etc. (z. B. gemäß WO 05/084081 oder WO 05/084082) oder der Atropisomere (z. B. gemäß der nicht offen gelegten Anmeldung EP 04026402.0). Besonders bevorzugte Hostmaterialien sind ausgewählt aus den Klassen der Oligo-arylene, enthaltend Naphthalin, Anthracen und/oder Pyren oder Atropisomeren dieser Verbindungen, der Oligo-arylenvinylene, der Ketone, der Phosphinoxide und der Sulfoxide. Ganz besonders bevorzugte Hostmaterialien sind ausgewählt aus den Klassen der Oligoarylene, enthaltend Anthracen und/oder Pyren oder Atropisomeren dieser Verbindungen, der Phosphinoxide und der Sulfoxide.

Der Anteil der Verbindung gemäß Formel (1) in der Mischung der emittierenden Schicht beträgt zwischen 0.1 und 99.0 Gew.%, bevorzugt zwischen 0.5 und 50.0 Gew.%, besonders bevorzugt zwischen 1.0 und 20.0 Gew.%, insbesondere zwischen 1.0 und 10.0 Gew.%. Entsprechend beträgt der Anteil des Hostmaterials in der Schicht zwischen 1.0 und 99.9 Gew.%, bevorzugt zwischen 50.0 und 99.5 Gew.%, besonders bevorzugt zwischen 80.0 und 99.0 Gew.%, insbesondere zwischen 90.0 und 99.0 Gew.%.

Es ist weiterhin bevorzugt, wenn die Verbindungen gemäß Formel (1) als Lochtransportmaterial und/oder Lochinjektionsmaterial eingesetzt werden, insbesondere in einer Lochtransportschicht und/oder in einer Lochinjektionsschicht. Dies gilt insbesondere dann, wenn das Symbol Y für N oder P steht. Hierbei kann es bevorzugt sein, wenn die Verbindung mit Elektronenakzeptor-Verbindungen dotiert ist, beispielsweise mit F₄-TCNQ oder mit Verbindungen, wie sie in EP 1476881 oder EP 1596445 beschrieben werden.

Es ist weiterhin bevorzugt, wenn die Verbindungen gemäß Formel (1) als Matrixmaterial für phosphoreszierende Emitter (Triplett-Emitter) eingesetzt werden. Dies gilt insbesondere dann, wenn das Symbol Y für C=O, P=O, S=O oder SO₂ steht.

Es ist weiterhin bevorzugt, wenn die Verbindungen gemäß Formel (1) als Elektronentransportmaterial, insbesondere in einer Elektronentransportschicht, und/oder als Lochblockiermaterial, insbesondere in einer Lochblockierschicht, in fluoreszierenden oder phosphoreszierenden Elektrolumineszenzvorrichtungen eingesetzt werden. Dies gilt insbesondere dann, wenn das Symbol Y für C=O, P=O, S=O oder SO₂ steht.

Auch in Polymeren können Verbindungen gemäß Formel (1) entweder als emittierende Einheit, als lochtransportierende Einheit, als elektronentransportierende Einheit oder als Matrix für phosphoreszierende Einheiten eingesetzt werden.

Wird die Verbindung gemäß Formel (1) als Lochtransportmaterial in einer Lochtransportschicht bzw. als Lochinjektionsmaterial in einer Lochinjektionsschicht bzw. als Elektronentransportmaterial in einer Elektronentransportschicht bzw. als Lochblockiermaterial in einer Lochblockierschicht eingesetzt, kann auch ein Anteil von 100 % bevorzugt sein, also die Verwendung dieser Verbindung als Reinmaterial.

Weiterhin bevorzugt sind organische Elektrolumineszenzvorrichtungen, dadurch gekennzeichnet, dass mehrere emittierende Verbindungen in derselben Schicht oder in unterschiedlichen Schichten verwendet werden, wobei mindestens eine dieser Verbindungen eine Struktur gemäß Formel (1) aufweist. Besonders bevorzugt weisen diese Verbindungen insgesamt mehrere Emissionsmaxima zwischen 380 nm und 750 nm auf, so dass insgesamt weiße Emission resultiert, d. h. außer der Verbindung gemäß Formel (1) wird noch mindestens eine weitere emittierende Verbindung verwendet, die fluoreszieren oder phosphoreszieren kann und die gelbes, orange oder rotes Licht emittiert. Insbesondere bevorzugt sind Dreischichtsysteme, wovon mindestens eine dieser Schichten eine Verbindung gemäß Formel (1) enthält und wobei die Schichten blaue, grüne und orange oder rote Emission zeigen (für den prinzipiellen Aufbau siehe z. B. WO 05/011013).

Außer Kathode, Anode und der emittierenden Schicht kann die organische Elektrolumineszenzvorrichtung noch weitere Schichten enthalten. Diese können beispielsweise sein: Lochinjektionsschicht, Lochtransportschicht, Elektronentransportschicht und/oder Elektroneninjektionsschicht. Es sei aber an dieser Stelle darauf hingewiesen, dass nicht notwendigerweise jede dieser Schichten vorhanden sein muss. So werden insbesondere bei Verwendung von Verbindungen gemäß Formel (1) mit elektronenleitenden Hostmaterialien weiterhin sehr gute Ergebnisse erhalten, wenn die organische Elektrolumineszenzvorrichtung keine separate Elektronentransportschicht enthält und die emittierende Schicht direkt an die Elektroneninjektionsschicht oder an die Kathode grenzt. Alternativ kann das Hostmaterial auch gleichzeitig in einer Elektronentransportschicht als Elektronentransportmaterial dienen. Ebenfalls kann es bevorzugt sein, wenn die organische Elektrolumineszenzvorrichtung keine separate Lochtransportschicht enthält und die emittierende Schicht direkt an die Lochinjektionsschicht oder an die Anode grenzt. Weiterhin kann es bevorzugt sein, wenn gleiche oder verschiedene Verbindungen gemäß Formel (1) gleichzeitig als Dotand in der emittierenden Schicht und als lochleitende Verbindung (als Reinsubstanz oder als Mischung) in einer Lochtransportschicht und/oder als elektronenleitende Verbindung in einer Elektronentransportschicht verwendet wird.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (1) nötig. Hohe Löslichkeit lässt sich durch geeignete Substitution der Verbindungen erreichen.

Die erfindungsgemäßen Verbindungen weisen bei Verwendung in organischen Elektrolumineszenzvorrichtungen folgende überraschende Vorteile gegenüber dem Stand der Technik auf:
1. Die Effizienz entsprechender Vorrichtungen wird höher im Vergleich zu Systemen gemäß dem Stand der Technik. Dies gilt insbesondere bei Verwendung von Verbindungen gemäß Formel (1) als Emitter für tiefblau emittierende Systeme.
2. Die Stabilität entsprechender Vorrichtungen wird höher im Vergleich zu Systemen gemäß dem Stand der Technik, was sich vor allem in einer deutlich höheren Lebensdauer zeigt.
3. Die Verbindungen lassen sich gut und ohne erhebliche Zersetzung sublimieren, sind dadurch leichter zu verarbeiten und deshalb besser für die Verwendung in OLEDs geeignet als Materialien gemäß dem Stand der Technik. Die höhere thermische Stabilität ist möglicherweise auf die Abwesenheit olefinischer Doppelbindungen zurückzuführen.
4. Werden die Verbindungen in einer Lochinjektions- bzw. Lochtransportschicht oder in einer Elektronentransportschicht eingesetzt, so zeigen die organischen Elektrolumineszenzvorrichtungen keine Abhängigkeit von der Schichtdicke der entsprechenden Schicht. Insbesondere bleiben die Betriebsspannung und die Leistungseffizienz auch bei großen Schichtdicken unverändert. Diese Eigenschaft ist von großer technischer Bedeutung für die Herstellung von Vollfarbdisplays.

Im vorliegenden Anmeldetext und auch in den im Weiteren folgenden Beispielen wird auf die Verwendung der erfindungsgemäßen Verbindungen in Bezug auf OLEDs und die entsprechenden Displays abgezielt. Trotz dieser Beschränkung der Beschreibung ist es für den Fachmann ohne weiteres erfinderisches Zutun möglich, die erfindungsgemäßen Verbindungen auch für weitere Verwendungen in anderen elektronischen Vorrichtungen einzusetzen, z. B. für organische Feld-Effekt-Transistoren (O-FETs), organische Dünnfilmtransistoren (O-TFTs), organische lichtemittierende Transistoren (O-LETs), organische integrierte Schaltungen (O-ICs), organische Solarzellen (O-SCs), organische Feld-Quench-Devices (O-FQDs), lichtemittierende elektrochemische Zellen (LECs), organische Photorezeptoren oder auch organische Laserdioden (O-Laser), um nur einige Anwendungen zu nennen. Die Verwendung der erfindungsgemäßen Verbindungen in den entsprechenden Vorrichtungen ebenso wie diese Vorrichtungen selbst sind ebenfalls ein Gegenstand der vorliegenden Erfindung.

Die Erfindung wird durch die nachfolgenden Beispiele näher erläutert, ohne sie dadurch einschränken zu wollen.

### Beispiele:

Die nachfolgenden Synthesen werden, sofern nicht anders angegeben, unter einer Schutzgasatmosphäre durchgeführt. Die Edukte wurden von der Firma ALDRICH (Palladium(II)acetat, Tri-tert-butylphosphin, Anorganika, Lösemittel) bezogen. 6,12-Dihydro-[1,2b]indenofluoren kann nach Hadizad et al., Org. Lett. 2005, 7(5), 795-797, [1,2b]Indenofluoren-6,12-dion nach Deuschel et al., Helv. Chimica Acta 1951, 34, 2403, 2-Brom-4,4'-di-tert-butylbiphenyl nach Tashiro et al., J. Org. Chem. 1979, 44(17), 3037 dargestellt werden.

### Beispiel 1: Tris-2-(8-ethyl-6,6,12,12-tetramethyl-6,12-dihydro-indeno[1,2b]fluoren-2-yl)amin

### a) 6,6,12,12-Tetramethyl-6,12-dihydro-indeno[1,2b]fluoren

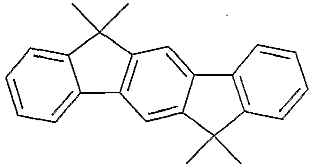

Die Darstellung erfolgt analog zur Darstellung von 9,9-Dimethylfluoren aus 6,12-Dihydro-indeno[1,2b]fluoren, Dimethylsulfat und Natronlauge nach JP 08113542. Ausbeute 86.0 % d. Th.; Reinheit 98 % n. ¹H-NMR.

### b) 2-Acetyl-6,6,12,12-tetramethyl-6,12-dihydro-indeno[1,2b]fluoren

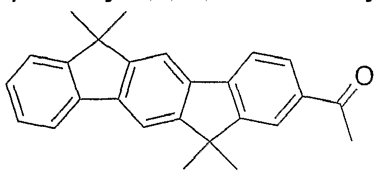

Eine Suspension von 16.0 g (120 mmol) Aluminiumchlorid in 500 ml 1,2-Dichlorethan wird tropfenweise mit 7.8 ml (110 mmol) Acetylchlorid versetzt. Zu dieser Mischung gibt man tropfenweise eine Lösung von 31.1 g (100 mmol) 6,6,12,12-Tetramethyl-6,12-dihydro-indeno[1,2b]fluoren in 500 ml 1,2-Dichlorethan. Anschließend rührt man 4 h bei Raumtemperatur nach, gießt die Mischung unter gutem Rühren in ein Gemisch aus 1000 g Eis und 200 ml 2 N Salzsäure ein und saugt vom ausgefallenen Feststoff ab. Der Feststoff wird dreimal mit 500 ml Wasser und dann dreimal mit 200 ml Ethanol gewaschen und im Vakuum getrocknet. Ausbeute: 31.1 g (88 mmol), 88.3 % d. Th.; Reinheit: 98 % n. ¹H-NMR.

### c) 2-Ethyl-6,6,12,12-tetramethyl-6,12-dihydro-indeno[1,2b]fluoren

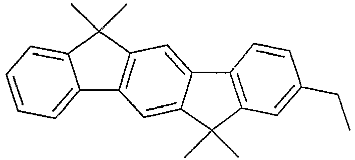

Eine Suspension von 28.2 g (80 mmol) 2-Acetyl-6,6,12,12-tetramethyl-6,12-dihydro-indeno[1,2b]fluoren in 300 ml Diethylenglykol wird mit 23.3 ml (480 mmol) Hydrazinhydrat versetzt und 24 h unter Rückfluss erhitzt. Nach Erkalten wird tropfenweise mit 300 ml 5 %igem Wasserstoffperoxid versetzt und 16 h bei Raumtemperatur gerührt. Der farblose Feststoff wird abgesaugt, dreimal mit 300 ml Wasser und dreimal mit 200 ml Ethanol gewaschen und im Vakuum getrocknet. Ausbeute: 25.9 g (76 mmol), 95.7 % d. Th.; Reinheit: 97 % n. ¹H-NMR.

### d) 2-Brom-8-ethyl-6,6,12,12-tetramethyl-6,12-dihydro-indeno[1,2b]fluoren

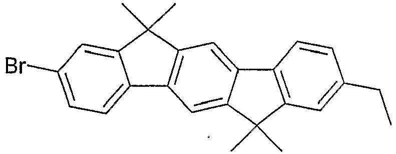

Eine Lösung von 10.2 g (30 mmol) 2-Ethyl-6,6,12,12-tetramethyl-6,12-dihydro-indeno[1,2b]fluoren in 300 ml Dichlormethan wird unter Lichtausschluss tropfenweise mit einem Gemisch aus 1.7 ml (32 mmol) Brom und 20 ml Dichlormethan versetzt. Nach 16 h Rühren bei Raumtemperatur gibt man 200 ml Ethanol und dann 50 ml gesättigte Natriumsulfitlösung zu. Der farblose Feststoff wird abgesaugt, dreimal mit 200 ml Wasser und dreimal mit 100 ml Ethanol gewaschen, im Vakuum getrocknet und dann zweimal aus DMF umkristallisiert. Ausbeute: 10.4 g (25 mmol), 83.0 % d. Th.; Reinheit: 97 % n. ¹H-NMR.

### e) 2-Ethyl-8-nitro-6,6,12,12-tetramethyl-6,12-dihydro-indeno[1,2b]fluoren

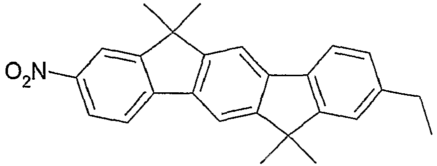

Eine gut gerührte, auf 0 °C gekühlte Suspension von 10.2 g (30 mmol) 2-Ethyl-6,6,12,12-tetramethyl-6,12-dihydro-indeno[1,2b]fluoren in 100 ml Dichlormethan wird unter Lichtausschluss tropfenweise mit einem Gemisch aus 4 ml 100 %ige Salpetersäure und 5 ml konz. Schwefelsäure versetzt. Nach 3 h Rühren bei Raumtemperatur gibt man 200 ml Wasser zu. Der gelbe Feststoff wird abgesaugt, dreimal mit 200 ml Wasser und dreimal mit 100 ml Ethanol gewaschen, im Vakuum getrocknet und dann zweimal aus o-Dichlorbenzol umkristallisiert. Ausbeute: 10.8 g (28 mmol), 93.7 % d. Th.; Reinheit: 98 % n. ¹H-NMR.

### f) 2-Amino-8-ethyl-6,6,12,12-tetramethyl-6,12-dihydro-indeno[1,2b]fluoren

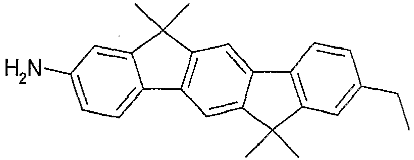

Eine gut gerührte, unter Rückfluss kochende Suspension von 9.6 g (25 mmol) 2-Ethyl-8-nitro-6,6,12,12-tetramethyl-6,12-dihydro-indeno[1,2b]fluoren in einem Gemisch von 100 ml Toluol und 200 ml Ethanol wird mit 4.9 ml (100 mmol) Hydrazinhydrat und dann mit 300 mg frisch bereitetem Raney-Nickel versetzt. Nach 2 h unter Rückfluss lässt man die Mischung erkalten, entfernt das Lösemittel im Vakuum, nimmt den Rückstand in 1000 ml warmem Chloroform auf, filtriert die Lösung über Kieselgel, engt die klare Lösung auf 100 ml ein und gibt 300 ml Ethanol zu. Nach 12 h stehen werden die farblosen Kristalle abgesaugt und anschließend zweimal aus Chloroform / Ethanol umkristallisiert. Ausbeute: 8.3 g (23.5 mmol), 93.9 % d. Th.; Reinheit: 98 % n. ¹H-NMR.

### g) Bis-2-[8-ethyl-6,6,12,12-tetramethyl-6,12-dihydro-indeno[1,2b] fluoren-8-yl]amin

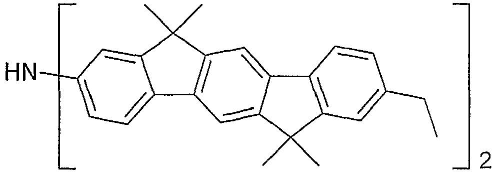

Eine Suspension von 7.1 g (20 mmol) 2-Amino-8-ethyl-6,6,12,12-tetramethyl-6,12-dihydro-indeno[1,2b]fluoren und 8.3 g (20 mmol) 2-Brom-8-ethyl-6,6,12,12-tetramethyl-6,12-dihydro-indeno[1,2b]fluoren in 250 ml Toluol wird mit 2.1 g (22 mmol) Natrium-tert-butylat, 55 mg (0.1 mmol) 1,1'-Diphenylphosphinoferrocen und 22 mg (0.1 mmol) Palladium(II)acetat versetzt und anschließend 16 h unter Rückfluss erhitzt. Nach Erkalten gibt man 250 ml Wasser zu, trennt die organische Phase ab, filtriert über Kieselgel und engt dann auf 30 ml ein. Nach Zugabe von 200 ml Ethanol und 16 h stehen werden die Kristalle abgesaugt und anschließend zweimal aus Chloroform / Ethanol umkristallisiert. Ausbeute: 9.9 g (14 mmol), 71.8 % d. Th.; Reinheit: 99 % n. ¹H-NMR.

### h) Tris-2-[8-ethyl-6,6,12,12-tetramethyl-6,12-dihydro-indeno[1,2b] fluoren-8-yl]amin

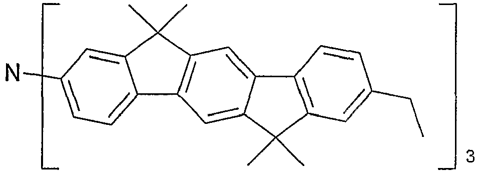

Eine Suspension von 6.9 g (10 mmol) Bis[2-ethyl-6,6,12,12-tetramethyl-6,12-dihydro-indeno[1,2b]fluoren-8-yl]amin und 4.2 g (10 mmol) 2-Brom-8-ethyl-6,6,12,12-tetramethyl-6,12-dihydro-indeno[1,2b]fluoren in 200 ml Toluol wird mit 1.2 g (12 mmol) Natrium-tert-butylat, 23.5 mg (0.13 mmol) Di-tert-butylchlorphosphin und 22.4 mg (0.1 mmol) Palladium(II)acetat versetzt und anschließend 16 h unter Rückfluss erhitzt. Nach Erkalten gibt man 300 ml Wasser zu, trennt die organische Phase ab, filtriert über Kieselgel und engt dann auf 30 ml ein. Nach Zugabe von 200 ml Ethanol und 16 h stehen, werden die Kristalle abgesaugt, anschließend siebenmal aus DMF umkristallisiert und dann im Vakuum (p = 1 x 10⁻⁵ mbar, T = 390 °C) sublimiert. Ausbeute: 5.3 g (5 mmol), 51.6 % d. Th.; Reinheit: 99.8 % n. ¹H-NMR.

### Beispiel 2: Bis-2-(8-ethyl-6,6,12,12-tetramethyl-6,12-dihydro-indeno[1,2b]fluoren-2-yl)keton

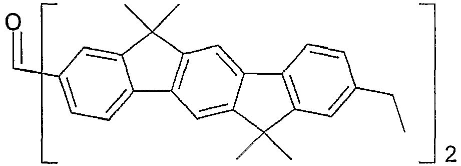

Eine auf -78 °C gekühlte Suspension von 8.4 g (20 mmol) 2-Brom-8-ethyl-6,6,12,12-tetramethyl-6,12-dihydro-indeno[1,2b]fluoren in 300 ml THF wird mit 27.3 ml (41 mmol) tert-BuLi (1.5 M in Hexan) versetzt und 8 h gerührt. Anschließend gibt man 0.9 ml (10 mmol) N,N-Dimethylcarbamoylchlorid zu, lässt auf Raumtemperatur erwärmen, rührt 16 h nach und gibt dann 10 ml Essigsäure und 20 ml Wasser zu. Nach Einengen der Reaktionsmischung im Vakuum nimmt man den Rückstand in 200 ml NMP auf, erhitzt unter Rückfluss, lässt auf 100 °C abkühlen, gibt 50 ml Wasser zu, lässt erkalten, saugt vom Feststoff ab und wäscht dreimal mit je 100 ml Ethanol nach. Anschließend wird fünfmal aus NMP umkristallisiert und dann im Vakuum (p = 1 x 10⁻⁵ mbar, T = 365 °C) sublimiert. Ausbeute: 4.8 g (7 mmol), 68.8 % d. Th.; Reinheit: 99.9 % n. ¹H-NMR.

### Beispiel 3: Tris-2-(8-ethyl-6,6,12,12-tetramethyl-6,12-dihydro-indeno[1,2b]fluoren-2-yl)phosphinoxid

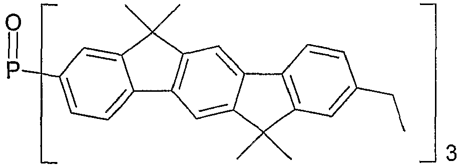

Eine auf -78 °C gekühlte Suspension von 8.8 g (21 mmol) 2-Brom-8-ethyl-6,6,12,12-tetramethyl-6,12-dihydro-indeno[1,2b]fluoren in 300 ml THF wird mit 28.7 ml (43 mmol) tert-BuLi, 1.5 M in Hexan versetzt und 8 h gerührt. Anschließend gibt man 0.6 ml (7 mmol) Phosphortrichlorid zu, lässt auf Raumtemperatur erwärmen, rührt 16 h nach und gibt dann 20 ml Wasser zu. Nach Einengen der Reaktionsmischung im Vakuum nimmt man den Rückstand in 200 ml Chloroform auf, gibt 0.9 ml (10 mmol) Wasserstoffperoxid 35 %ig und 20 ml Wasser zu. Man erhitzt das Gemisch 5 h auf 60 °C, lässt erkalten, saugt vom Feststoff ab und wäscht dreimal mit je 100 ml Wasser und dreimal mit je 100 ml Ethanol nach. Anschließend wird fünfmal aus DMF umkristallisiert und dann im Vakuum (p = 1 x 10⁻⁵ mbar, T = 395 °C) sublimiert. Ausbeute: 5.4 g (5 mmol), 72.9 % d. Th.; Reinheit: 99.9 % n. ¹H-NMR.

### Beispiel 4: 2-(Di(4-methylphenyl)amino)-8-ethyl-dispiro[2,7-di-tert-butyl-fluoren-9,6'-indenofluoren-[1,2b]fluoren-12',9"-fluoren]

### a) Dispiro[2,7-di-tert-butyl-fluoren-9,6'-indenofluoren-[1,2b]-fluoren-12",9" -fluoren]

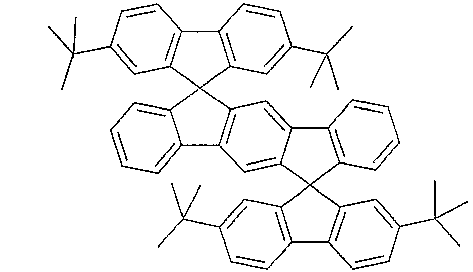

Aus 6.2 g (255 mmol) Magnesium und 86.3 g (250 mmol) 2-Brom-4,4'-di-tert-butylbiphenyl in 500 ml THF wird das entsprechende Grignard-Reagenz dargestellt. Zu diesem Grignard-Reagenz gibt man weitere 500 ml THF und 28.8 g (100 mmol) [1,2b]Indenofluoren-6,12-dion. Die Reaktionsmischung wird 10 h unter Rückfluss erhitzt, nach Erkalten mit 50 ml Ethanol versetzt und im Vakuum zur Trockene eingeengt. Der Rückstand wird in einem Gemisch aus 1000 ml Essigsäure und 25 ml konz. Salzsäure 3 h unter Rückfluss erhitzt. Nach Erkalten werden die farblosen Kristalle abgesaugt, mit 100 ml Essigsäure, dann dreimal mit je 100 ml Ethanol gewaschen und im Vakuum getrocknet. Anschließend wird zweimal aus Chlorbenzol umkristallisiert. Ausbeute: 56.9 g (73 mmol), 73.0 % d. Th.; Reinheit: 99 % n. ¹H-NMR.

### b) 2-Acetyl-dispiro[2,7-di-tert-butyl-fluoren-9,6'-indenofluoren-[1,2b] fluoren-12',9" -fluoren]

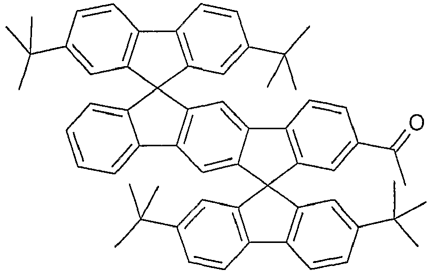

Durchführung analog Beispiel 1b. Anstelle von 6,6,12,12-Tetramethyl-6,12-dihydro-indeno[1,2b]fluoren werden 77.9 g (100 mmol) Dispiro[2,7-di-tert-butyl-fluoren-9,6'-indenofluoren-[1,2b]fluoren-12',9"-fluoren] eingesetzt. Aufarbeitung: Man gießt die Mischung unter gutem Rühren in ein Gemisch aus 1000 g Eis und 200 ml 2 N Salzsäure ein, trennt die organische Phase ab, wäscht diese dreimal mit 500 ml Wasser und engt im Vakuum ein. Der Feststoff wird mit 500 ml Ethanol heiß ausgerührt, abgesaugt, mit 100 ml Ethanol gewaschen und im Vakuum getrocknet. Ausbeute: 65.0 g (79 mmol), 79.2 % d. Th.; Reinheit: 98 % n. ¹H-NMR.

### c) 2-Ethyl-dispiro[2,7-di-tert-butyl-fluoren-9,6'-indenofluoren-[1,2b] fluoren-12',9"-fluoren

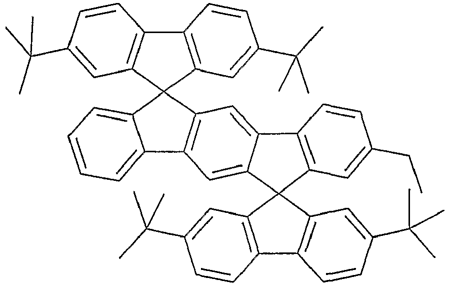

Durchführung analog Beispiel 1c. Anstelle von 2-Acetyl-6,6,12,12-tetramethyl-6,12-dihydro-indeno[1,2b]fluoren werden 65.8 g (80 mmol) 2-Acetyl-dispiro[2,7-di-tert-butyl-fluoren-9,6'-indenofluoren-[1,2b]fluoren-12',9"-fluoren] eingesetzt. Ausbeute: 62.2 g (77 mmol), 96.3 % d. Th.; Reinheit: 98 % n. ¹H-NMR.

### d) 2-Brom-8-ethyl-dispiro[2,7-di-tert-butyl-fluoren-9,6'-indenofluoren-[1,2b]fluoren-12',9"-fluoren]

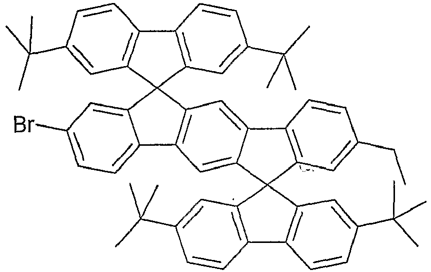

Durchführung analog Beispiel 1c. Anstelle von 2-Ethyl-6,6,12,12-tetramethyl-6,12-dihydro-indeno[1,2b]fluoren werden 24.2 g (30 mmol) 2-Ethyl-dispiro[2,7-di-tert-butyl-fluoren-9,6'-indenofluoren-[1,2b]fluoren-12',9"-fluoren] eingesetzt. Die Umkristallisation erfolgt aus Chlorbenzol. Ausbeute: 23.3 g (26 mmol), 87.8 % d. Th.; Reinheit: 98 % n. ¹H-NMR.

### e) 2-(Di(4-methylphenyl)amino)-8-ethyl-dispiro[2,7-di-tert-butyl-fluoren-9,6'-indenofluoren-[1,2b]fiuoren-12',9"-fluoren]

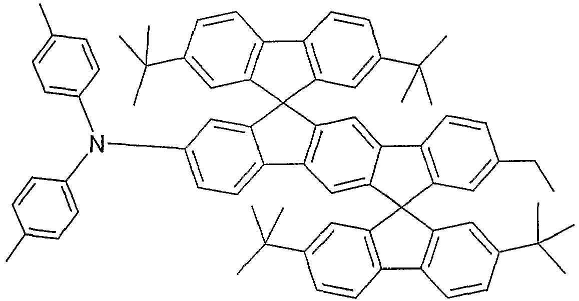

Durchführung analog Beispiel 1g. Anstelle von 2-Brom-8-ethyl-6,6,12,12-tetramethyl-6,12-dihydro-indeno[1,2b]fluoren werden 8.7 g (10 mmol) 2-Brom-8-ethyl-dispiro[2,7-di-tert-butyl-fluoren-9,6'-indenofluoren-[1,2b]fluoren-12',9"-fluoren], anstelle von Bis[2-ethyl-6,6,12,12-tetramethyl-6,12-dihydro-indeno[1,2b]fluoren-8-yl]amin werden 2.0 g (10 mmol) Bis(4-methylphenyl)amin eingesetzt. Die Umkristallisation erfolgt aus Chlorbenzol, anschließend wird im Vakuum (p = 1 x 10⁻⁵ mbar, T = 410 °C) sublimiert. Ausbeute: 6.0 g (6 mmol), 59.7 % d. Th; Reinheit: 99.9 % n. ¹H-NMR.

### Beispiel 5: Tris-2-(8-ethyl-6,6,12,12-tetramethyl-6,12-dihydro-cis-indenofluoren-2-yl)amin

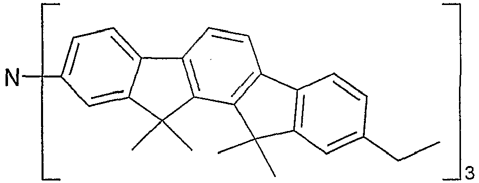

Tris-2-(8-ethyl-6,6,12,12-tetramethy)-6,12-dihydro-cis-indenofluoren-2-yl)amin lässt sich in Analogie zu Beispiel 1 synthetisieren, wobei sich cis-Indenofluoren aus Ausgangsverbindung gemäß WO 04/113412 synthetisieren lässt.

### Beispiel 6: Bis-2-(8-ethyl-6,6,12,12-tetramethyl-6,12-dihydro-cis-indenofluoren-2-yl)keton

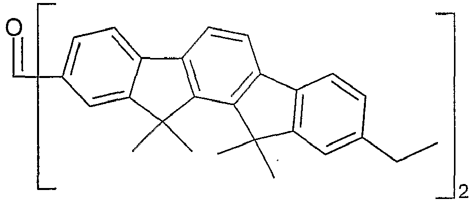

Bis-2-(8-ethyl-6,6,12,12-tetramethyl-6,12-dihydro-cis-indenofluoren-2-yl)keton lässt sich in Analogie zu Beispiel 2 synthetisieren, wobei sich cis-Indenofluoren aus Ausgangsverbindung gemäß WO 04/113412 synthetisieren lässt.

### Beispiel 7: Tris-2-(8-ethyl-6,6,12,12-tetramethyl-6,12-dihydro-cis-indenofluoren-2-yl)phosphinoxid

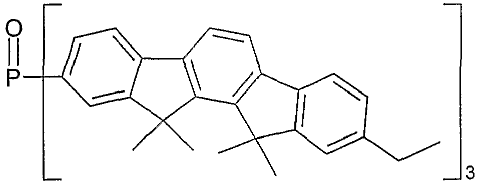

Tris-2-(8-ethy)-6,6,12,12-tetramethyl-6,12-dihydro-cis-indenofluoren-2-yl)phosphinoxid lässt sich in Analogie zu Beispiel 3 synthetisieren, wobei sich cis-Indenofluoren aus Ausgangsverbindung gemäß WO 04/113412 synthetisieren lässt.

### Beispiel 8: Herstellung von OLEDs mit Tris-2-(8-ethyl-6,6,12,12-tetramethyl-6,12-dihydro-indeno[1,2b]fluoren-2-yl)amin

Die Herstellung von OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen 9 bis 15 werden die Ergebnisse verschiedener OLEDs vorgestellt. Der grundlegende Aufbau und die verwendeten Materialien (außer der Lochtransportschicht) sind in den Beispielen zur besseren Vergleichbarkeit identisch. Analog dem o. g. allgemeinen Verfahren werden OLEDs mit folgendem Aufbau erzeugt:
- Lochinjektionsschicht (HIL): 20 nm PEDOT (aus Wasser aufgeschleudert; bezogen von H. C. Starck, Goslar, Deutschland; Poly(3,4-ethylendioxy-2,5-thiophen))
- Lochtransportschicht (HTM): Variable Schichtdicke, s. Tabelle 1, Tris-2-(8-ethyl-6,6,12,12-tetramethyl-6,12-dihydro-indeno[1,2b]fluoren-2-yl)amin (abgekürzt als **HTM-1,** aufgedampft, synthetisiert nach Beispiel 1);
- ODER:: als Vergleichsbeispiel 4,4',4"-Tris(N-1-naphthyl-N-phenyl-amino)-triphenylamin (abgekürzt als NaphDATA bezogen von SynTec)
- Lochtransportschicht (HTM): 30 nm NPB (N-Naphthyl-N-phenyl-4,4'-diaminobiphenyl)
- Emissionschicht (EML): 30 nm, dotierte Schicht aus 9,10-Bis(1-naphthylanthracen) als Hostmaterial (abgekürzt **H**), dotiert mit 5 % Tris[4-(2,2-diphenyl-vinyl)-phenyl]amin als Dotand (abgekürzt **D**, aufgedampft, synthetisiert nach WO 06/000388)
- Elektronenleiter (ETL): 20 nm AlQ₃ (bezogen von SynTec, Tris(chinolinato)aluminium(III))
- Kathode: 1 nm LiF, darauf 150 nm Al.

Diese OLEDs werden standardmäßig charakterisiert; hierfür werden die Elektrolumineszenzspektren, die Effizienz (gemessen in cd/A) und die Leistungseffizienz (gemessen in Im/W) in Abhängigkeit der Helligkeit, berechnet aus Strom-Spannungs-Helligkeit-Kennlinien (IUL-Kennlinien), bestimmt.

In Tabelle 1 sind die Ergebnisse einiger OLEDs (Beispiele 9 bis 15), bei denen die Schichtdicke der Lochtransportschicht (HTM) aus Tris-2-(8-ethyl-6,6,12,12-tetramethyl-6,12-dihydro-indeno[1,2b]fluoren-2-yl)amin variiert wird, zusammengefasst. Als Vergleichsmaterial wird in den Vergleichsbeispielen NaphDATA verwendet.
Das Hostmaterial H ist 9,10-Bis(1-naphthyl)anthracen, als Dotand wird **D** eingesetzt. Beide sind im Folgenden dargestellt:

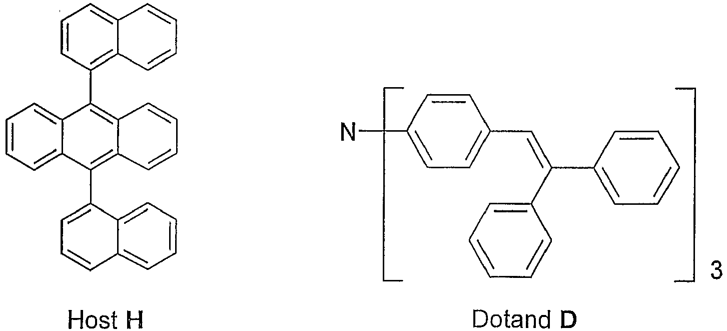

Wie man den erfindungsgemäßen Beispielen 11 bis 15 in der Tabelle 1 entnehmen kann, zeigen OLEDs enthaltend das erfindungsgemäße Lochtransportmaterial Tris-2-(8-ethyl-6,6,12,12-tetramethyl-6,12-dihydro-indeno[1,2b]fluoren-2-yl)amin eine deutlich niedrigere Betriebsspannung als mit NaphDATA gemäß dem Stand der Technik als Lochtransportmaterial. Weiterhin ist die Betriebsspannung unabhängig von der Schichtdicke der Lochtransportschicht. Diese Eigenschaft ist für den Aufbau vollfarbiger Displays von großem Vorteil, da man durch Variation der Schichtdicke der Lochtransportschicht die Dicke der Pixel der Grundfarben Blau, Grün und Rot gleich gestalten kann. Das erfindungsgemäße Lochtransportmaterial dient hier also aus Dickenausgleichsschicht, ohne dass dadurch die elektooptischen Eigenschaften des Devices nachteilig beeinflusst werden. Wie den Vergleichsbeispielen zu entnehmen ist, ist dies für ein Lochstransportmaterial (NaphDATA) gemäß Stand der Technik nicht der Fall: Hier wird bei größerer Schichtdicke der Lochtransportschicht eine deutlich höhere Betriebsspannung benötigt.

**Tabelle 1**

| **Beispiel** | **HTL** | **Max. Effizienz (cd/A)** | **Spannung (V) bei 1000cd/m²** | **CIE** |
|---|---|---|---|---|
| Beispiel 9 (Vergleich) | NaphDATA (30 nm) | 7.9 | 6.6 | x=0.17; y=0.31 |
| Beispiel 10 (Vergleich) | NaphDATA (100 nm) | 7.3 | 7.7 | x=0.16; y=0.30 |
| Beispiel 11 | **HTM-1** (30 nm) | 7.9 | 5.8 | x=0.16; y=0.30 |
| Beispiel 12 | **HTM-1** (50 nm) | 7.9 | 5.8 | x=0.16; y=0.30 |
| Beispiel 13 | **HTM-1** (100 nm) | 8.1 | 5.7 | x=0.16; y=0.30 |
| Beispiel 14 | **HTM-1** (150 nm) | 8.0 | 5.9 | x=0.16; =0.29 |
| Beispiel 15 | **HTM-1** (200 nm) | 7.8 | 6.0 | x=0.16; y=0.30 |

### Beispiel 16: Herstellung von phosphoreszierenden OLEDs mit Bis-2-(8-ethyl-6,6,12,12-tetramethyl-6,12-dihydro-indeno[1,2b]-fluoren-2-yl)keton und Tris-2-(8-ethyl-6,6,12,12-tetramethyl-6,12-dihydro-indeno[1,2b]fluoren-2-yl)phosphinoxid

Die Herstellung von OLEDs erfolgt nach einem allgemeinen Verfahren gemäß WO 04/093207, das im Einzelfall auf die jeweiligen Gegebenheiten (z. B. Schichtdickenvariation, um optimale Effizienz bzw. Farbe zu erreichen) angepasst wird.

Hier werden die Ergebnisse verschiedener OLEDs gegenübergestellt. Der grundlegende Aufbau, wie die verwendeten Materialien, Dotierungsgrad und ihre Schichtdicken ist für die Beispielexperimente zur besseren Vergleichbarkeit identisch. Es wurde ausschließlich das Matrixmaterial in der Emitterschicht getauscht, und die Beispiele werden mit unterschiedlichen Triplett-Emittern durchgeführt.

Beispiel 17 und 18 beschreiben Vergleichsstandards nach dem Stand der Technik, bei dem die Emitterschicht als Matrixmaterial CBP bzw. ein Keton (abgekürzt als **Keton-1**) enthält. Des Weiteren werden OLEDs mit einer Emitterschicht bestehend aus den erfindungsgemäßen Emittermaterialien Bis-2-(8-ethyl-6,6,12,12-tetramethyl-6,12-dihydro-indeno[1,2b]fluoren-2-yl)keton und Tris-2-(8-ethyl-6,6,12,12-tetramethyl-6,12-dihydro-indeno[1,2b]fluoren-2-yl)phosphinoxid beschrieben.

Analog dem o. g. allgemeinen Verfahren werden OLEDs mit folgendem Aufbau erzeugt:
- PEDOT: 60 nm (aus Wasser aufgeschleudert; PEDOT bezogen von H. C. Starck; Poly-[3,4-ethylendioxy-2,5-thiophen])
- NaphDATA: 20 nm (aufgedampft; NaphDATA bezogen von SynTec; 4,4',4"-Tris(N-1-naphthyl-N-phenyl-amino)-triphenylamin
- S-TAD: 20 nm (aufgedampft; S-TAD hergestellt nach WO99/12888; 2,2',7,7'-Tetrakis(diphenylamino)-spirobifluoren)
- Emitter-Schicht:: CBP (aufgedampft; CBP bezogen von ALDRICH und weiter gereinigt, schließlich noch zweimal sublimiert; 4,4'-Bis-(N-carbazolyl)biphenyl) (Vergleichsstandard)
- ODER:: **Keton-1** (Bis(9,9'-spirobifluoren-2-yl)keton (aufgedampft, synthetisiert nach WO 04/093207) (Vergleichsstandard)
- ODER:: Bis-2-(8-ethyl-6,6,12,12-tetramethyl-6,12-dihydro-indeno[1,2b]fluoren-2-yl)keton (abgekürzt **M1,** aufgedampft, synthetisiert nach Beispiel 2),
- ODER:: Tris-2-(8-ethyl-6,6,12,12-tetramethyl-6,12-dihydro-indeno[1,2b]fluoren-2-yl)phosphinoxid (abgekürzt **M2**, aufgedampft, synthetisiert nach Beispiel 3),
jeweils dotiert mit 10 % Triplett-Emitter **E1** (synthetisiert nach WO 05/033244), **E2** (synthetisiert nach US 2003/0068526) oder **E3** (synthetisiert nach US 2003/0068526)
Bathocuproin
- (BCP): 10 nm (aufgedampft; BCP bezogen von ABCR, verwendet wie erhalten; 2,9-Dimethyl-4,7-diphenyl-1,10-phenanthrolin); nicht in allen Beispielen verwendet
- AlQ₃: 10 nm (aufgedampft; AlQ₃ bezogen von SynTec; Tris(chinolinolato)aluminium(III)), nicht in allen Beispielen verwendet
- Ba-Al: 3 nm Ba, darauf 150 nm Al als Kathode.

Diese OLEDs werden standardmäßig charakterisiert; hierfür werden die Elektrolumineszenzspektren, die Effizienz (gemessen in cd/A), die Leistungseffizienz (gemessen in Im/W) in Abhängigkeit der Helligkeit, berechnet aus Strom-Spannungs-Helligkeit-Kennlinien (IUL-Kennlinien), und die Lebensdauer bestimmt. Als Lebensdauer wird die Zeit definiert, nach der die Anfangshelligkeit von 1000 cd/m² auf die Hälfte gesunken ist. Zur Übersicht sind im Folgenden die verwendeten Triplett-Emitter und CBP und **Keton-1** als Vergleichsmaterialien abgebildet:

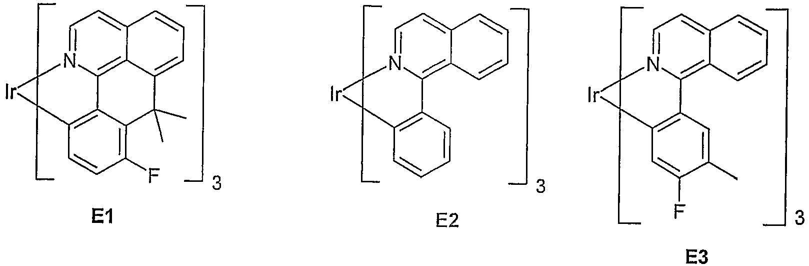

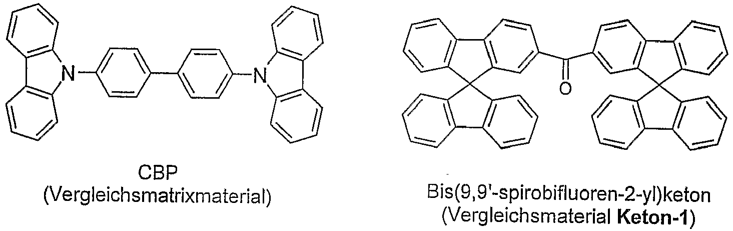

**Tabelle 2:**

| Experiment | EML | HBL | ETL | Max. Effizienz (cd/A) | Max. Leistungseffizienz (Im/W) | x, y (CIE) | Lebensdauer (h) bei 1000 cd/cm² |
|---|---|---|---|---|---|---|---|
| Beispiel 17 (Vergleich) | CBP: | BCP (10 nm) | AlQ₃ (10 nm) | 11.5 | 6.3 | 0.64, 0.36 | 2000 (extrapoliert) |
| | 15% **E1** | | | | | | |
| | (30 nm) | | | | | | |
| Beispiel 18 (Vergleich) | Keton-1: | BCP (10 nm) | AlQ₃ (10 nm) | 10.3 | 7.5 | 0.64, 0.36 | 12700 (extrapoliert) |
| | 15% **E1** | | | | | | |
| | (30 nm) | | | | | | |
| Beispiel 19 | **M1:** | BCP (10 nm) | AlQ₃ (10 nm) | 18.2 | 11.5 | 0.65, 0.35 | 14000 (extrapoliert) |
| | 15% **E1** | | | | | | |
| | (30 nm) | | | | | | |
| Beispiel 20 | **M2:** | BCP (10 nm) | AlQ₃ (10 nm) | 18.7 | 12.3 | 0.64, 0.36 | 12000 (extrapoliert) |
| | 15% **E1** | | | | | | |
| | (30 nm) | | | | | | |
| Beispiel 21 | **M1:** | --- | AlQ₃ (10 nm) | 21.7 | 14.3 | 0.65, 0.35 | 13500 (extrapoliert) |
| | 15% **E1** | | | | | | |
| | (30 nm) | | | | | | |
| Beispiel 22 | **M2:** | --- | AlQ₃ (10 nm) | 20.9 | 14.8 | 0.64, 0.36 | 11500 (extrapoliert) |
| | 15% **E1** | | | | | | |
| | (30 nm) | | | | | | |
| Beispiel 23 | **M1:** | --- | --- | 23.0 | 15.7 | 0.65, 0.35 | 6000 (extrapoliert) |
| | 15% **E1** | | | | | | |
| | (30 nm) | | | | | | |
| Beispiel 24 | **M2:** | --- | --- | 23.2 | 17.1 | 0.64, 0.36 | 5500 (extrapoliert) |
| | **15% E1** | | | | | | |
| | (30 nm) | | | | | | |
| Beispiel 25 (Vergleich) | CBP: | BCP (10 nm) | AlQ₃ (10 nm) | 6.3 | 4.7 | 0.68, 0.32 | 5000 (extrapoliert) |
| | 15 % **E2** | | | | | | |
| | (20 nm) | | | | | | |
| Beispiel 26 (Vergleich) | **Keton-1:** : | BCP (10 nm) | AlQ₃ (10 nm) | 8.0 | 6.0 | 0.68, 0.32 | 20000 (extrapoliert) |
| | 15 % **E2** | | | | | | |
| | (20 nm) | | | | | | |
| Beispiel 27 | M1: | BCP (10 nm) | AlQ₃ (10 nm) | 8.8 | 7.7 | 0.69, 0.31 | 22000 (extrapoliert) |
| | 15 % **E2** | | | | | | |
| | (20 nm) | | | | | | |
| Beispiel 28 | **M2**: | BCP (10 nm) | AlQ₃ (10 nm) | 8.5 | 7.9 | 0.68, 0.32 | 21000 (extrapoliert) |
| | 15 % **E2** | | | | | | |
| | (20 nm) | | | | | | |
| Beispiel 29 (Vergleich) | CBP: | BCP (10 nm) | AlQ₃ (10 nm) | 10.3 | 7.3 | 0.66, 0.34 | 4000 (extrapoliert) |
| | 15 % **E3** | | | | | | |
| | (20 nm) | | | | | | |
| Beispiel 30 | **M1:** | BCP (10 nm) | AlQ₃ (10 nm) | 14.6 | 12.3 | 0.67, 0.33 | 15000 (extrapoliert) |
| | 15 % **E3** | | | | | | |
| | (20 nm) | | | | | | |
| Beispiel 31 | **M2:** | BCP (10 nm) | AlQ₃ (10 nm) | 15.7 | 12.6 | 0.66, 0.34 | 14500 (extrapoliert) |
| | 15 % **E3** | | | | | | |
| | (20 nm) | | | | | | |

### Elektrolumineszenzspektren:

Die OLEDs, sowohl die Vergleichsbeispiele als auch die OLEDs mit **M1** bzw. **M2** als Matrixmaterial zeigen rote Emission mit vergleichbaren Farbkoordinaten.

### Effizienz:

Mit OLEDs hergestellt mit den erfindungsgemäßen Matrixmaterialien **M1** bzw. **M2** zeigen sowohl deutliche besser photometrische Effizienz als auch bessere Leistungseffizienzen im Vergleich zu Matrixmaterialien gemäß dem Stand der Technik. Dies gilt insbesondere für die in anwendungstechnischer Hinsicht maßgebliche Leistungseffizienz, bedingt durch die geringeren Betriebsspannungen bei Verwendung der Matrixmaterialien **M1** bzw. **M2.**

### Lebensdauer:

Die Lebensdauer, die bei durch Verwendung der erfindungsgemäßen Matrixmaterialen **M1** bzw. M2 erreicht wird, übertrifft die der Vergleichsbeispiele mit dem Matrixmaterial CBP erheblich und übertrifft auch die Vergleichsbeispiele mit dem Matrixmaterial **Keton-1.**

### Schichtvereinfachung:

Wie den Beispielen 20 bis 23 entnommen werden kann, ist es mit den erfindungsgemäßen Matrixmaterialien **M1** und **M2** möglich, OLEDs, die weder eine Lochblockierschicht noch eine Elekronenleiterschicht enthalten herzustellen, ohne dass dadurch das elektrooptische Gesamteigenschaftsprofil verschlechtert wird. Dies ist produktionstechnisch ein erheblicher Vorteil, der bei Verwendung von Matrixmaterialen gemäß dem Stand der Technik nicht realisiert werden kann.

### Beispiel 32: Herstellung von fluoreszierenden OLEDs mit Bis-2-(8-ethyl-6,6,12,12-tetramethyl-6,12-dihydro-indeno[1,2b]-fluoren-2-yl)keton und Tris-2-(8-ethyl-6,6,12,12-tetramethyl-6,12-dihydro-indeno[1,2b]fluoren-2-yl)phosphinoxid

Die Herstellung von OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen 33 bis 38 werden die Ergebnisse verschiedener OLEDs vorgestellt. Der grundlegende Aufbau und die verwendeten Materialien (außer der Elektronentransportschicht) sind in den Beispielen zur besseren Vergleichbarkeit identisch. Analog dem o. g. allgemeinen Verfahren werden OLEDs mit folgendem Aufbau erzeugt:
- Lochinjektionsschicht (HIL): 20 nm PEDOT (aus Wasser aufgeschleudert; bezogen von H. C. Starck, Goslar, Deutschland; Poly(3,4-ethylendioxy-2,5-thiophen))
- Lochtransportschicht (HTM): 50 nm, Tris-2-(8-ethyl-6,6,12,12-tetramethyl-6,12-dihydro-indeno[1,2b]-fluoren-2-yl)amin (abgekürzt als **HTM-1,** aufgedampft, synthetisiert nach Beispiel 1)
- Lochtransportschicht (HTM): 30 nm NPB (N-Naphthyl-N-phenyl-4,4'-diaminobiphenyl)
- Emissionschicht (EML): 30 nm, dotierte Schicht aus 9,10-Bis(1-naphthylanthracen) als Hostmaterial (abgekürzt H), dotiert mit 5 % Tris[4-(2,2-diphenyl-vinyl)-phenyl]amin als Dotand (abgekürzt **D,** aufgedampft, synthetisiert nach WO 06/000388)
- Elektronenleiter (ETL): Bis-2-(8-ethyl-6,6,12,12-tetramethyl-6,12-dihydro-indeno[1,2b]fluoren-2-yl)keton (abgekürzt **EL1**, aufgedampft, synthetisiert nach Beispiel 2),
- ODER:: Bis-2-(8-ethyl-6,6,12,12-tetramethyl-6,12-dihydro-indeno[1,2b]fluoren-2-yl)phosphin-oxid, (abgekürzt **EL2,** aufgedampft, synthetisiert nach Beispiel 3),
- ODER:: 10 nm (aufgedampft; AlQ₃ bezogen von SynTec; Tris(chinolinolato)-aluminium(III))
- Kathode: 1 nm LiF, darauf 150 nm Al.

Diese OLEDs werden standardmäßig charakterisiert; hierfür werden die Elektrolumineszenzspektren, die Effizienz (gemessen in cd/A) und die Leistungseffizienz (gemessen in Im/W) in Abhängigkeit der Helligkeit, berechnet aus Strom-Spannungs-Helligkeit-Kennlinien (IUL-Kennlinien), bestimmt.

In Tabelle 3 sind die Ergebnisse einiger OLEDs (Beispiele 35 bis 38), bei denen die Elektronentransportschicht (ETM) aus Bis-2-(8-ethyl-6,6,12,12-tetramethyl-6,12-dihydro-indeno[1,2b]fluoren-2-yl)keton bzw. Tris-2-(8-ethyl-6,6,12,12-tetramethyl-6,12-dihydro-indeno[1,2b]fluoren-2-yl)phosphinoxid besteht, zusammengefasst. Als Vergleichsmaterial wird in den Vergleichsbeispielen AlQ₃ gemäß dem Stand der Technik verwendet.

Wie man den Beispielen 33 bis 38 in der Tabelle 3 entnehmen kann, zeigen OLED-Devices enthaltend die erfindungsgemäßen Elektronentransportmaterialien Bis-2-(8-ethyl-6,6,12,12-tetramethyl-6,12-dihydro-indeno[1,2b]fluoren-2-yl)keton bzw. Tris-2-(8-ethyl-6,6,12,12-tetramethyl-6,12-dihydro-indeno[1,2b]fluoren-2-yl)phosphinoxid eine von der Schichtdicke unabhängige Betriebsspannung. Diese Eigenschaft ist für den Aufbau vollfarbiger Displays von großem Vorteil, da man durch Variation der Schichtdicke der Elektronentransportschicht die Dicke der Pixel der Grundfarben Blau, Grün und Rot gleich gestalten kann. Das erfindungsgemäße Elektronentransportmaterial dient hier also aus Dickenausgleichsschicht, ohne dass dadurch die elektooptischen Eigenschaften des Devices nachteilig beeinflusst werden. Wie den Vergleichsbeispielen zu entnehmen ist, ist dies für ein Elektronentransportmaterial AlQ₃ gemäß dem Stand der Technik nicht der Fall.

**Tabelle 3**

| **Beispiel** | **ETL** | **Max Effizienz (cd/A)** | **Spannung (V) bei 1000cd/m²** | **CIE** |
|---|---|---|---|---|
| Beispiel 33 (Vergleich) | AlQ₃ | 7.9 | 5.8 | x=0.16; |
| | (10 nm) | | | y=0.30 |
| Beispiel 34 (Vergleich) | AlQ₃ | 6.2 | 6.6 | x=0.16; |
| | (30 nm) | | | y=0.33 |
| Beispiel 35 | **EL-1** | 7.8 | 5.4 | x=0.16; |
| | (10 nm) | | | y=0.27 |
| Beispiel 36 | **EL-1** | 8.2 | 5.5 | x=0.16; |
| | (30 nm) | | | y=0.27 |
| Beispiel 37 | **EL-2** | 8.0 | 5.2 | x=0.16; |
| | (10 nm) | | | y=0.27 |
| Beispiel 38 | **EL-2** | 8.3 | 5.3 | x=0.16; |
| | (30 nm) | | | y=0.28 |

### Beispiel 39: Herstellung von OLEDs mit 2-(Di(4-methylphenyl)amino)-8-ethyl-dispiro[2,7-di-tert-butyl-fluoren-9,6'-indenofluoren-[1,2b]fluoren-12',9"-fluoren]

Die Herstellung von OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen 40 bis 42 werden die Ergebnisse verschiedener OLEDs vorgestellt. Der grundlegende Aufbau und die verwendeten Materialien (außer der emittierenden Schicht) sind in den Beispielen zur besseren Vergleichbarkeit identisch. Analog dem o. g. allgemeinen Verfahren werden OLEDs mit folgendem Aufbau erzeugt:
- Lochinjektionsschicht (HIL): 20 nm PEDOT (aus Wasser aufgeschleudert; bezogen von H. C. Starck, Goslar, Deutschland; Poly(3,4-ethylendioxy-2,5-thiophen))
- Lochtransportschicht (HTM): 50 nm Tris-2-(8-ethyl-6,6,12,12-tetramethyl-6,12-dihydro-indeno[1,2b]-fluoren-2-yl)amin (abgekürzt als **HTM-1,** aufgedampft, synthetisiert nach Beispiel 1)
- Lochtransportschicht (HTM): 30 nm NPB (N-Naphthyl-N-phenyl-4,4'-diaminobiphenyl)
- Emissionschicht (EML): 30 nm, dotierte Schicht aus 9,10-Bis(1-naphthylanthracen) als Hostmaterial (abgekürzt **H**), dotiert mit x %, s. Tabelle, 2-(Di(4-methylphenyl)amino)-8-ethyl-dispiro[2,7-di-tert-butyl-fluoren-9,6'-indenofluoren-[1,2b]fluoren-12',9"-fluoren] als Dotand (abgekürzt **D**, aufgedampft, synthetisiert nach Beispiel 4)
- Elektronenleiter (ETL): 20 nm Bis-2-(8-ethyl-6,6,12,12-tetra-methyl-6,12-dihydro-indeno[1,2b]fluoren-2-yl)keton (abgekürzt **EL1,** aufgedampft, synthetisiert nach Beispiel 2),
- Kathode: 1 nm LiF, darauf 150 nm AI.

Diese OLEDs werden standardmäßig charakterisiert; hierfür werden die Elektrolumineszenzspektren, die Effizienz (gemessen in cd/A) und die Leistungseffizienz (gemessen in Im/W) in Abhängigkeit der Helligkeit, berechnet aus Strom-Spannungs-Helligkeit-Kennlinien (IUL-Kennlinien), bestimmt.

In Tabelle 4 sind die Ergebnisse einiger OLEDs (Beispiele 40 bis 42), bei denen 2-(Di(4-methylphenyl)amino)-8-ethyl-dispiro[2,7-di-tert-butyl-fluoren-9,6'-indenofluoren-[1,2b]fluoren-12',9"-fluoren] als tiefblauer Emitter verwendet und dessen Dotierungsgrad variiert wird, zusammengefasst. Das Hostmaterial **H** ist 9,10-Bis(1-naphthyl)anthracen, ist im Folgenden dargestellt:

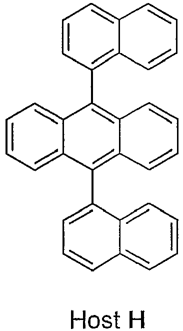

Wie man den Beispielen 40 bis 42 in der Tabelle 1 entnehmen kann, zeigen OLEDs enthaltend den erfindungsgemäßen Dotanden 2-(Di(4-methylphenyl)amino)-8-ethyl-dispiro[2,7-di-tert-butyl-fluoren-9,6'-indenofluoren-[1,2b]fluoren-12',9"-fluoren] effiziente tiefblaue Emission.

**Tabelle 4**

| **Beispiel** | **EML** | **Max Effizienz (cd/A)** | **Spannung (V) bei 1000cd/m²** | **CIE** |
|---|---|---|---|---|
| Beispiel 40 | **H** | 2.3 | 6.8 | x=0.10; |
| | 2 % **D** | | | y=0.05 |
| Beispiel 41 | **H** | 2.4 | 6.7 | x=0.10; |
| | 5 % **D** | | | y=0.07 |
| Beispiel 42 | **H** | 2.6 | 6.5 | x=0.11; |
| | 10 % **D** | | | y=0.08 |

## Patentansprüche

1. Verbindungen gemäß Formel (1), 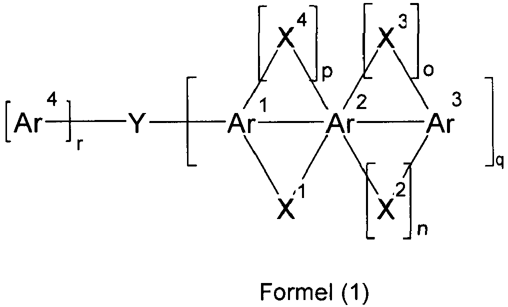 wobei für die verwendeten Symbole und Indizes gilt:
Y ist bei jedem Auftreten N, P, P=O, PF₂, P=S, As, As=O, As=S, Sb, Sb=O, Sb=S, C=O, O, S, Se, Te, S=O, SO₂, Se=O, SeO₂, Te=O oder TeO₂;
Ar¹, Ar², Ar³ ist Benzol, das jeweils mit einem oder zwei Resten R¹ substituiert sein kann;
Ar⁴ ist bei jedem Auftreten gleich oder verschieden ein aromatisches oder heteroaromatisches Ringsystem mit 5 bis 40 aromatischen Ringatomen, das mit einem oder mehreren Resten R¹ substituiert sein kann;
R¹ ist bei jedem Auftreten gleich oder verschieden H, F, Cl, Br, I, CN, NO₂, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch -R²C=CR²-, -C≡C-, Si(R²)₂, Ge(R²)2, Sn(R²)2, C=O, C=S, C=Se, C=NR², -O-, -S- oder -CONR²- ersetzt sein können und wobei ein oder mehrere H-Atome durch F, Cl, Br, I, CN oder NO₂ ersetzt sein können, oder ein aromatisches oder heteroaromatisches Ringsystem mit 5 bis 40 aromatischen Ringatomen, das durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann, oder eine Kombination dieser Systeme; dabei können zwei oder mehrere Substituenten R¹ auch miteinander ein mono- oder polycyclisches aliphatisches Ringsystem bilden;
R² ist bei jedem Auftreten gleich oder verschieden H oder ein aliphatischer oder aromatischer Kohlenwasserstoffrest mit 1 bis 20 C-Atomen;
X¹, X⁴ ist bei jedem Auftreten gleich oder verschieden eine Brücke, die mit Ar¹ und Ar² ein cyclisches System aufspannt, ausgewählt aus C(R¹)₂, Si(R¹)₂, C=O, C=NR¹, C=C(R¹)2, O, S, S=O, SO₂, N(R¹), P(R¹), P(=O)R¹, P(=S)R¹, B(R¹) oder einer Kombination aus zwei, drei oder vier dieser Gruppen;
X², X³ ist bei jedem Auftreten gleich oder verschieden X¹ und spannt mit Ar² und Ar³ ein cyclisches Ringsystem auf;
n, o, p ist bei jedem Auftreten gleich oder verschieden 0 oder 1, mit der Maßgabe für Y aus der fünften Hauptgruppe, dass n, p und o nur dann gleichzeitig 0 sein können, wenn q = 3 ist; dabei bedeutet n = 0 bzw. o = 0 bzw. p = 0, dass sich an Stelle der Brücke zwei H-Atome oder Reste R¹ befinden;
q ist 1, 2 oder 3, wenn Y über ein Element der fünften Hauptgruppe gebunden ist, und ist 2, wenn Y über Sauerstoff gebunden ist, und ist 1 oder 2, wenn Y über ein anderes Element der sechsten Hauptgruppe gebunden ist;
r ist (3 - q), wenn Y über ein Element der fünften Hauptgruppe gebunden ist, und ist (2 - q), wenn Y über ein anderes Element der sechsten Hauptgruppe gebunden ist.

2. Verbindungen gemäß Anspruch 1, **dadurch gekennzeichnet, dass** das Symbol Y für Stickstoff, C=O, Phosphor oder P=O steht.

3. Verbindungen gemäß Anspruch 1 oder 2 gemäß Formel (2), 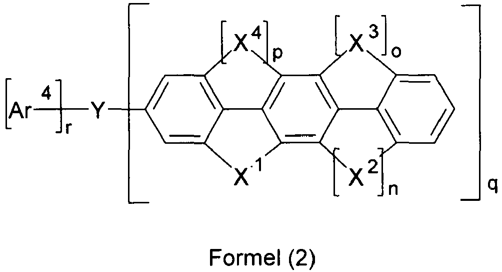 wobei die verwendeten Symbole und Indizes dieselbe Bedeutung haben, wie in Anspruch 1 beschrieben, und wobei jede der Phenyl- bzw. Phenylengruppen noch durch einen oder mehrere Reste R¹ substituiert sein kann.

4. Verbindungen gemäß einem oder mehreren der Ansprüche 1 bis 3, **dadurch gekennzeichnet, dass** das Symbol Ar⁴ gleich oder verschieden bei jedem Auftreten für ein aromatisches oder heteroaromatisches Ringsystem mit 5 bis 16 aromatischen Ringatomen oder für Spirobifluoren steht, das jeweils mit einem oder mehreren Resten R¹ substituiert sein kann.

5. Verbindungen gemäß einem oder mehreren der Ansprüche 1 bis 4, **dadurch gekennzeichnet, dass** das Symbol R¹ gleich oder verschieden bei jedem Auftreten für H, F, CN, Si(R²)₃, eine geradkettige Alkylgruppe mit 1 bis 5 C-Atomen oder eine verzweigte Alkylgruppe mit 3 bis 5 C-Atomen, wobei jeweils eine oder mehrere nicht benachbarte CH₂-Gruppen durch Si(R²)₂, -R²C=CR²-, -C=C-, -O- oder -S- ersetzt sein können und wobei ein oder mehrere H-Atome durch F ersetzt sein können, oder eine monovalente Aryl- oder Heteroarylgruppe mit 2 bis 16 C-Atomen, die mit einem oder mehreren Resten R² substituiert sein kann, steht.

6. Verbindungen gemäß einem oder mehreren der Ansprüche 1 bis 5, **dadurch gekennzeichnet, dass** die Symbole X¹, X², X³ und X⁴ bei jedem Auftreten gleich oder verschieden eine Brücke darstellen, die mit Ar¹ und Ar² bzw. mit Ar² und Ar³ ein cyclisches System aufspannt, ausgewählt aus C(R¹)₂, C=O, C=NR¹, O, S, S=O, SO₂, N(R¹), P(R¹), P(=O)R¹, C(R¹)₂-C(R¹)₂, C(R¹)₂-C(R¹)₂-C(R¹)₂, C(R¹)₂-O, C(R¹)₂-O-C(R¹)₂.

7. Verbindungen gemäß einem oder mehreren der Ansprüche 1 bis 6, **dadurch gekennzeichnet, dass** der Index p = 0 ist und einer der beiden Indizes n oder o = 1 ist, während der andere der beiden Indizes = 0 ist.

8. Verbindungen gemäß einem oder mehreren der Ansprüche 1 bis 7, **dadurch gekennzeichnet, dass** der Index q bei jedem Auftreten für 2 oder 3 steht für Y aus der fünften Hauptgruppe und dass der Index q bei jedem Auftreten für 2 steht für Y aus der sechsten Hauptgruppe.

9. Verbindungen gemäß einem oder mehreren der Ansprüche 1 bis 8, **dadurch gekennzeichnet, dass** die Verbindungen symmetrisch aufgebaut sind und die eine dreizählige Drehachse aufweisen für Y aus der fünften Hauptgruppe und eine zweizählige Drehachse für Y aus der sechsten Hauptgruppe.

10. Konjugierte, teilkonjugierte oder nicht-konjugierte Polymere, Oligomere oder Dendrimere, enthaltend eine oder mehrere Verbindungen gemäß Formel (1), wobei eine oder mehrere Bindungen der Verbindung gemäß Formel (1) zum Polymer, Oligomer oder Dendrimer vorhanden sind.

11. Polymere, Oligomere oder Dendrimere gemäß Anspruch 10, **dadurch gekennzeichnet, dass** das Polymergrundgerüst ausgewählt ist aus der Gruppe bestehend aus Polyfluorenen, Poly-spirobifluorenen, Poly-paraphenylenen, Polycarbazolen, Polyvinylcarbazolen, Polythiophenen, Polydihydrophenanthrenen, Polyindenofluorenen, Polyketonen oder Copolymeren enthaltend mehrere dieser Einheiten.

12. Mischungen, enthaltend mindestens eine Verbindung gemäß einem oder mehreren der Ansprüche 1 bis 9 und mindestens ein Hostmaterial.

13. Verwendung von Verbindungen oder Mischungen gemäß einem oder mehreren der Ansprüche 1 bis 12 in organischen elektronischen Vorrichtungen.

14. Organische elektronische Vorrichtung, enthaltend mindestens eine Verbindung oder Mischung gemäß einem oder mehreren der Ansprüche 1 bis 12.

15. Organische elektronische Vorrichtung gemäß Anspruch 14, ausgewählt aus der Gruppe bestehend aus organischen Elektrolumineszenzvorrichtungen (OLEDs), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Photorezeptoren oder organischen Laserdioden (O-Laser).

16. Organische Elektrolumineszenzvorrichtung, enthaltend Anode, Kathode und mindestens eine emittierende Schicht, **dadurch gekennzeichnet, dass** die emittierende Schicht mindestens eine Verbindung oder Mischung gemäß einem oder mehreren der Ansprüche 1 bis 12 enthält.

17. Organische Elektrolumineszenzvorrichtung gemäß Anspruch 16, **dadurch gekennzeichnet, dass** das Hostmaterial ausgewählt ist aus den Klassen der Oligo-arylene, der Oligo-arylene enthaltend kondensierte aromatische Gruppen, der Oligo-arylenvinylene, der polypodalen Metallkomplexe, der lochleitenden Verbindungen, der elektronenleitenden Verbindungen, der Ketone, der Phosphinoxide, der Sulfoxide oder der Atropisomere.

18. Organische Elektrolumineszenzvorrichtung gemäß Anspruch 16 oder 17, **dadurch gekennzeichnet, dass** der Anteil der Verbindung gemäß einem oder mehreren der Ansprüche 1 bis 11 in der Mischung der emittierenden Schicht zwischen 0.5 und 50.0 Gew.% beträgt und der Anteil des Hostmaterials entsprechend zwischen 50.0 und 99.5 Gew.% beträgt.

19. Organische elektronische Vorrichtung gemäß einem oder mehreren der Ansprüche 14 bis 18, **dadurch gekennzeichnet, dass** weitere Schichten vorhanden sind, ausgewählt aus Lochinjektionsschicht, Lochtransportschicht, Elektronentransportschicht und/oder Elektroneninjektionsschicht.

20. Organische Elektrolumineszenzvorrichtung, enthaltend Anode, Kathode und mindestens eine emittierende Schicht, **dadurch gekennzeichnet**, mindestens eine Lochtransportschicht und/oder mindestens eine Lochinjektionsschicht vorhanden ist, welche mindestens eine Verbindung gemäß einem oder mehreren der Ansprüche 1 bis 11 enthält.

21. Organische Elektrolumineszenzvorrichtung, enthaltend Anode, Kathode und mindestens eine emittierende Schicht, welche mindestens einen phosphoreszierenden Emitter enthält, **dadurch gekennzeichnet, dass** die emittierende Schicht als Matrixmaterial mindestens eine Verbindung gemäß einem oder mehreren der Ansprüche 1 bis 11 enthält.

22. Organische Elektrolumineszenzvorrichtung, enthaltend Anode, Kathode und mindestens eine emittierende Schicht, **dadurch gekennzeichnet, dass** mindestens eine Elektronentransportschicht und/oder mindestens eine Lochblockierschicht vorhanden ist, welche mindestens eine Verbindung gemäß einem oder mehreren der Ansprüche 1 bis 11 enthält.

## Claims

1. Compounds of the formula (1), 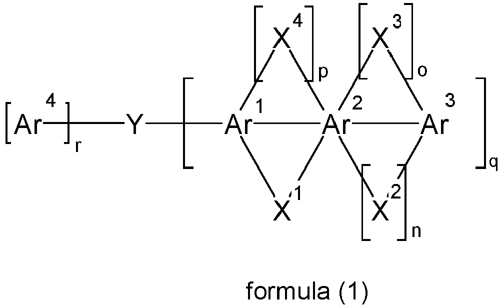 where the following applies to the symbols and indices used:
Y is on each occurrence N, P, P=O, PF₂, P=S, As, As=O, As=S, Sb, Sb=O, Sb=S, C=O, O, S, Se, Te, S=O, SO₂, Se=O, SeO₂, Te=O or TeO₂;
Ar¹, Ar², Ar³ are benzene, which may in each case be substituted by one or two radicals R¹;
Ar⁴ is on each occurrence, identically or differently, an aromatic or heteroaromatic ring system having 5 to 40 aromatic ring atoms, which may be substituted by one or more radicals R¹;
R¹ is on each occurrence, identically or differently, H, F, Cl, Br, I, CN, NO₂, Si(R²)₃, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals R², where one or more non-adjacent CH₂ groups may be replaced by -R²C=CR²-, -C≡C-, Si(R²)₂, Ge(R²)₂, Sn(R²)₂, C=O, C=S, C=Se, C=NR², -O-, -S- or -CONR²- and where one or more H atoms may be replaced by F, Cl, Br, I, CN or NO₂, or an aromatic or heteroaromatic ring system having 5 to 40 aromatic ring atoms, which may be substituted by one or more radicals R², or an aryloxy or heteroaryloxy group having 5 to 40 aromatic ring atoms, which may be substituted by one or more radicals R², or a combination of these systems; two or more substituents R¹ may also form a mono- or polycyclic aliphatic ring system with one another;
R² is on each occurrence, identically or differently, H or an aliphatic or aromatic hydrocarbon radical having 1 to 20 C atoms;
X¹, X⁴ are on each occurrence, identically or differently, a bridge which, with Ar¹ and Ar², forms a cyclic system, selected from C(R¹)₂, Si(R¹)₂, C=O, C=NR¹, C=C(R¹)₂, O, S, S=O, SO₂, N(R¹), P(R¹), P(=O)R¹, P(=S)R¹, B(R¹) or a combination of two, three or four of these groups;
X², X³ are on each occurrence, identically or differently, X¹ and, with Ar² and Ar³, form a cyclic ring system;
n, o, p are on each occurrence, identically or differently, 0 or 1, with the proviso that, if Y is from the fifth main group, n, p and o may only simultaneously be 0 if q = 3; n = 0 or o = 0 or p = 0 means that two H atoms or radicals R¹ are present instead of the bridge;
q is 1, 2 or 3 if Y is bonded via an element from the fifth main group, and is 2 if Y is bonded via oxygen, and is 1 or 2 if Y is bonded via another element from the sixth main group;
r is (3 - q) if Y is bonded via an element from the fifth main group, and is (2 - q) if Y is bonded via another element from the sixth main group.

2. Compounds according to Claim 1, **characterised in that** the symbol Y stands for nitrogen, C=O, phosphorus or P=O.

3. Compounds according to Claim 1 or 2 of the formula (2), 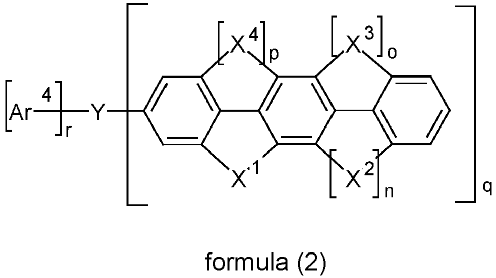 where the symbols and indices used have the same meaning as described in Claim 1, and where each of the phenyl and phenylene groups may also be substituted by one or more radicals R¹.

4. Compounds according to one or more of Claims 1 to 3, **characterised in that** the symbol Ar⁴ stands, identically or differently on each occurrence, for an aromatic or heteroaromatic ring system having 5 to 16 aromatic ring atoms or for spirobifluorene, which may in each case be substituted by one or more radicals R¹.

5. Compounds according to one or more of Claims 1 to 4, **characterised in that** the symbol R¹ stands, identically or differently on each occurrence, for H, F, CN, Si(R²)₃, a straight-chain alkyl group having 1 to 5 C atoms or a branched alkyl group having 3 to 5 C atoms, where in each case one or more non-adjacent CH₂ groups may be replaced by Si(R²)₂, -R²C=CR²-, -C≡C-, -O- or -S- and where one or more H atoms may be replaced by F, or a monovalent aryl or heteroaryl group having 2 to 16 C atoms, which may be substituted by one or more radicals R².

6. Compounds according to one or more of Claims 1 to 5, **characterised in that** the symbols X¹, X², X³ and X⁴ on each occurrence, identically or differently, represent a bridge which, with Ar¹ and Ar² or with Ar² and Ar³, forms a cyclic system selected from C(R¹)₂, C=O, C=NR¹, O, S, S=O, SO₂, N(R¹), P(R¹), P(=O)R¹, C(R¹)₂-C(R¹)₂, C(R¹)₂-C(R¹)₂-C(R¹)₂, C(R¹)₂-O, C(R¹)2-O-C(R¹)2.

7. Compounds according to one or more of Claims 1 to 6, **characterised in that** the index p = 0 and one of the two indices n and o = 1, while the other of the two indices = 0.

8. Compounds according to one or more of Claims 1 to 7, **characterised in that** the index q stands on each occurrence for 2 or 3 if Y is from the fifth main group and **in that** the index q stands on each occurrence for 2 if Y is from the sixth main group.

9. Compounds according to one or more of Claims 1 to 8, **characterised in that** the compounds have a symmetrical structure and a three-fold axis of rotational symmetry if Y is from the fifth main group and a twofold axis of rotational symmetry if Y is from the sixth main group.

10. Conjugated, partially conjugated or non-conjugated polymers, oligomers or dendrimers comprising one or more compounds of the formula (1), where one or more bonds are present from the compound of the formula (1) to the polymer, oligomer or dendrimer.

11. Polymers, oligomers or dendrimers according to Claim 10, **characterised in that** the polymer skeleton is selected from the group consisting of polyfluorenes, polyspirobifluorenes, poly-para-phenylenes, polycarbazoles, polyvinylcarbazoles, polythiophenes, polydihydrophen-anthrenes, polyindenofluorenes, polyketones or copolymers comprising a plurality of these units.

12. Mixtures comprising at least one compound according to one or more of Claims 1 to 9 and at least one host material.

13. Use of compounds or mixtures according to one or more of Claims 1 to 12 in organic electronic devices.

14. Organic electronic device containing at least one compound or mixture according to one or more of Claims 1 to 12.

15. Organic electronic device according to Claim 14, selected from the group consisting of organic electroluminescent devices (OLEDs), organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs), light-emitting electrochemical cells (LECs), organic photoreceptors and organic laser diodes (O-lasers).

16. Organic electroluminescent device comprising anode, cathode and at least one emitting layer, **characterised in that** the emitting layer comprises at least one compound or mixture according to one or more of Claims 1 to 12.

17. Organic electroluminescent device according to Claim 16, **characterised in that** the host material is selected from the classes of the oligoarylenes, the oligoarylenes containing condensed aromatic groups, the oligoarylenevinylenes, the polypodal metal complexes, the hole-conducting compounds, the electron-conducting compounds, the ketones, the phosphine oxides, the sulfoxides or the atropisomers.

18. Organic electroluminescent device according to Claim 16 or 17, **characterised in that** the proportion of the compound according to one or more of Claims 1 to 11 in the mixture of the emitting layer is between 0.5 and 50.0% by weight, and the proportion of the host material is correspondingly between 50.0 and 99.5% by weight.

19. Organic electronic device according to one or more of Claims 14 to 18, **characterised in that** further layers selected from hole-injection layer, hole-transport layer, electron-transport layer and/or electron-injection layer are present.

20. Organic electroluminescent device comprising anode, cathode and at least one emitting layer, **characterised in that** at least one hole-transport layer and/or at least one hole-injection layer which comprises at least one compound according to one or more of Claims 1 to 11 is present.

21. Organic electroluminescent device comprising anode, cathode and at least one emitting layer which comprises at least one phosphorescent emitter, **characterised in that** the emitting layer comprises, as matrix material, at least one compound according to one or more of Claims 1 to 11.

22. Organic electroluminescent device comprising anode, cathode and at least one emitting layer, **characterised in that** at least one electron-transport layer and/or at least one hole-blocking layer which comprises at least one compound according to one or more of Claims 1 to 11 is present.

## Revendications

1. Composés de la formule (1), 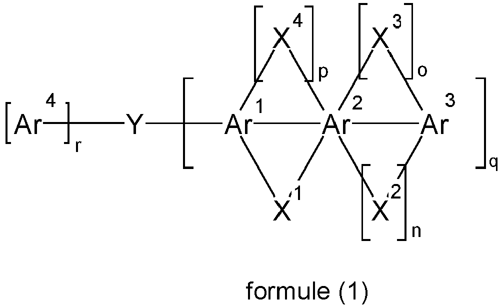 dans laquelle ce qui suit s'applique aux symboles et aux indices qui sont utilisés :
Y est pour chaque occurrence N, P, P=O, PF₂, P=S, As, As=O, As=S, Sb, Sb=O, Sb=S, C=O, O, S, Se, Te, S=O, SO₂, Se=O, SeO₂, Te=O ou TeO₂ ;
Ar¹, Ar², Ar³ sont benzène, lequel peut dans chaque cas être substitué par un radical ou par deux radicaux R¹ ;
Ar⁴ est pour chaque occurrence, de manière identique ou différente, un système de cycle aromatique ou hétéroaromatique qui comporte de 5 à 40 atomes de cycle aromatique, lequel peut être substitué par un radical ou par plusieurs radicaux R¹ ;
R¹ est pour chaque occurrence, de manière identique ou différente, H, F, Cl, Br, I, CN, NO₂, Si(R²)₃, un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes de C, lequel peut dans chaque cas être substitué par un radical ou par plusieurs radicaux R², où un ou plusieurs groupe(s) CH₂ non adjacents peut/ peuvent être remplacé(s) par -R²C=CR²-, -C≡C-, Si(R²)₂, Ge(R²)₂, Sn(R²)₂, C=O, C=S, C=Se, C=NR², -O-, -S- ou -CONR²- et où un ou plusieurs atome(s) de H peut/peuvent être remplacé(s) par F, Cl, Br, I, CN ou NO₂, ou un système de cycle aromatique ou hétéroaromatique qui comporte de 5 à 40 atomes de cycle aromatique, lequel peut être substitué par un radical ou par plusieurs radicaux R², ou un groupe aryloxy ou hétéroaryloxy qui comporte de 5 à 40 atomes de cycle aromatique, lequel peut être substitué par un radical ou par plusieurs radicaux R², ou une combinaison de ces systèmes ; deux substituants R¹ ou plus peuvent également former un système de cycle aliphatique mono- ou polycyclique l'un avec l'autre ou les uns avec les autres ;
R² est pour chaque occurrence, de manière identique ou différente, H ou un radical hydrocarbone aliphatique ou aromatique qui comporte de 1 à 20 atome(s) de C ;
X¹, X⁴ sont pour chaque occurrence, de manière identique ou différente, un pont qui, avec Ar¹ et Ar², forme un système cyclique, sélectionné parmi C(R¹)₂, Si(R¹)₂, C=O, C=NR¹, C=C(R¹)₂, O, S, S=O, SO₂, N(R¹), P(R¹), P(=O)R¹, P(=S)R¹, B(R¹) ou une combinaison de deux, trois ou quatre de ces groupes ;
X², X³ sont pour chaque occurrence, de manière identique ou différente, X¹ et, avec Ar² et Ar³, forment un système de cycle cyclique ;
n, o, p sont pour chaque occurrence, de manière identique ou différente, 0 ou 1, étant entendu que, si Y provient du cinquième groupe principal, n, p et o peuvent seulement simultanément être 0 si q = 3 ; n = 0 ou o = 0 ou p = 0 signifie que deux atomes de H ou deux radicaux R¹ sont présents en lieu et place du pont ;
q est 1, 2 ou 3 si Y est lié via un élément qui provient du cinquième groupe principal, et est 2 si Y est lié via oxygène, et est 1 ou 2 si Y est lié via un autre élément qui provient du sixième groupe principal ;
r est (3 - q) si Y est lié via un élément qui provient du cinquième groupe principal, et est (2 - q) si Y est lié via un autre élément qui provient du sixième groupe principal.

2. Composés selon la revendication 1, **caractérisés en ce que** le symbole Y représente azote, C=O, phosphore ou P=O.

3. Composés selon la revendication 1 ou 2 de la formule (2), 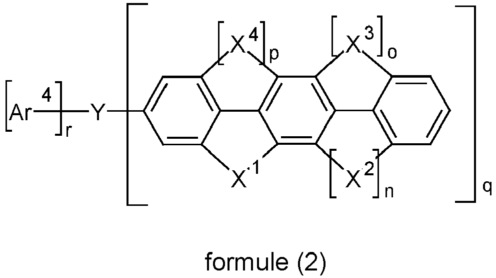 dans laquelle les symboles et les indices qui sont utilisés présentent la même signification que décrit selon la revendication 1, et où chacun des groupes phényle et phénylène peut également être substitué par un radical ou par plusieurs radicaux R¹.

4. Composés selon une ou plusieurs des revendications 1 à 3, **caractérisés en ce que** le symbole Ar⁴ représente, de manière identique ou différente pour chaque occurrence, un système de cycle aromatique ou hétéroaromatique qui comporte de 5 à 16 atomes de cycle aromatique ou spirobifluorène, lequel peut dans chaque cas être substitué par un radical ou par plusieurs radicaux R¹.

5. Composés selon une ou plusieurs des revendications 1 à 4, **caractérisés en ce que** le symbole R¹ représente, de manière identique ou différente pour chaque occurrence, H, F, CN, Si(R²)₃, un groupe alkyle en chaîne droite qui comporte de 1 à 5 atome(s) de C ou un groupe alkyle ramifié qui comporte de 3 à 5 atomes de C, où dans chaque cas, un ou plusieurs groupe(s) CH₂ non adjacents peut/peuvent être remplacé(s) par Si(R²)₂, -R²C=CR²-, -C≡C-, -O- ou -S- et où un ou plusieurs atome(s) de H peut/peuvent être remplacé(s) par F, ou un groupe aryle ou hétéroaryle monovalent qui comporte de 2 à 16 atomes de C, lequel peut être substitué par un radical ou par plusieurs radicaux R².

6. Composés selon une ou plusieurs des revendications 1 à 5, **caractérisés en ce que** les symboles X¹, X², X³ et X⁴ représentent pour chaque occurrence, de manière identique ou différente, un pont qui, avec Ar¹ et Ar² ou avec Ar² et Ar³, forme un système cyclique qui est sélectionné parmi C(R¹)₂, C=O, C=NR¹, O, S, S=O, SO₂, N(R¹), P(R¹), P(=O)R¹, C(R¹)₂-C(R¹)₂, C(R¹)₂-C(R¹)₂-C(R¹)₂, C(R¹)₂-O, C(R¹)2-O-C(R¹)2.

7. Composés selon une ou plusieurs des revendications 1 à 6, **caractérisés en ce que** l'indice p = 0 et l'un des deux indices n et o = 1, tandis que l'autre des deux indices = 0.

8. Composés selon une ou plusieurs des revendications 1 à 7, **caractérisés en ce que** l'indice q représente pour chaque occurrence 2 ou 3 si Y provient du cinquième groupe principal et **en ce que** l'indice q représente pour chaque occurrence 2 si Y provient du sixième groupe principal.

9. Composés selon une ou plusieurs des revendications 1 à 8, **caractérisés en ce que** les composés présentent une structure symétrique et un axe de symétrie de rotation d'ordre trois si Y provient du cinquième groupe principal et un axe de symétrie de rotation d'ordre deux si Y provient du sixième groupe principal.

10. Polymères, oligomères ou dendrimères conjugués, partiellement conjugués ou non conjugués comprenant un ou plusieurs composé(s) de la formule (1), où une ou plusieurs liaison(s) est/sont présente(s) depuis le composé de la formule (1) jusqu'au polymère, à l'oligomère ou au dendrimère.

11. Polymères, oligomères ou dendrimères selon la revendication 10, **caractérisés en ce que** le squelette de polymère est sélectionné parmi le groupe qui est constitué par les polyfluorènes, les polyspiro-bifluorènes, les poly-para-phénylènes, les polycarbazoles, les polyvinylcarbazoles, les polythiophènes, les polydihydrophénanthrènes, les polyindénofluorènes, les polycétones ou les copolymères qui comprennent une pluralité de ces unités.

12. Mélanges comprenant au moins un composé selon une ou plusieurs des revendications 1 à 9 et au moins un matériau hôte.

13. Utilisation de composés ou de mélanges selon une ou plusieurs des revendications 1 à 12 dans les dispositifs électroniques organiques.

14. Dispositif électronique organique contenant au moins un composé ou un mélange selon une ou plusieurs des revendications 1 à 12.

15. Dispositif électronique organique selon la revendication 14, sélectionné parmi le groupe qui est constitué par les dispositifs électroluminescents organiques (OLED), les transistors à effet de champ organiques (O-FET), les transistors à film mince organiques (O-TFT), les transistors à émission de lumière organiques (O-LET), les circuits intégrés organiques (O-IC), les cellules solaires organiques (O-SC), les dispositifs à extinction de champ organiques (O-FQD), les cellules électrochimiques à émission de lumière (LEC), les photorécepteurs organiques et les diodes laser organiques (O-laser).

16. Dispositif électroluminescent organique comprenant une anode, une cathode et au moins une couche d'émission, **caractérisé en ce que** la couche d'émission comprend au moins un composé ou un mélange selon une ou plusieurs des revendications 1 à 12.

17. Dispositif électroluminescent organique selon la revendication 16, **caractérisé en ce que** le matériau hôte est sélectionné parmi les classes des oligoarylènes, les oligoarylènes contenant des groupes aromatiques condensés, les oligoarylènevinylènes, les complexes métalliques polypodaux, les composés de conduction de trous, les composés de conduction d'électrons, les cétones, les oxydes de phosphine, les sulfoxydes ou les atropisomères.

18. Dispositif électroluminescent organique selon la revendication 16 ou 17, **caractérisé en ce que** la proportion du composé selon une ou plusieurs des revendications 1 à 11 dans le mélange de la couche d'émission se situe entre 0,5 % et 50,0 % en poids, et la proportion du matériau hôte se situe en correspondance entre 50,0 % et 99,5 % en poids.

19. Dispositif électronique organique selon une ou plusieurs des revendications 14 à 18, **caractérisé en ce que** d'autres couches qui sont sélectionnées parmi une couche d'injection de trous, une couche de transport de trous, une couche de transport d'électrons et/ou une couche d'injection d'électrons sont présentes.

20. Dispositif électroluminescent organique comprenant une anode, une cathode et au moins une couche d'émission, **caractérisé en ce qu'**au moins une couche de transport de trous et/ou au moins une couche d'injection de trous qui comprennent/comprend au moins un composé selon une ou plusieurs des revendications 1 à 11 sont/est présente(s).

21. Dispositif électroluminescent organique comprenant une anode, une cathode et au moins une couche d'émission qui comprend au moins un émetteur phosphorescent, **caractérisé en ce que** la couche d'émission comprend, en tant que matériau de matrice, au moins un composé selon une ou plusieurs des revendications 1 à 11.

22. Dispositif électroluminescent organique comprenant une anode, une cathode et au moins une couche d'émission, **caractérisé en ce qu'**au moins une couche de transport d'électrons et/ou au moins une couche de blocage de trous qui comprennent/comprend au moins un composé selon une ou plusieurs des revendications 1 à 11 sont/est présente(s).
